# FASCICULE DE BREVET EUROPEEN

(11) **EP 0 119 895 B1**
(45) Date de publication et mention de la délivrance du brevet: **28.01.1987**
(21) Numéro de dépôt: 84400367.3
(22) Date de dépôt: 23.02.1984
(51) Int. Cl.: C07D 339/04, C07D 409/12, A61K 31/385

(54) **Nouveaux dérivés de la dithiole-1,2 one-3, leur préparation et les compositions médicinales qui les contiennent**
1,2-Dithiol-3-on-Derivate, ihre Herstellung und diese enthaltende medizinische Zusammenstellungen
1,2-Dithiol-3-one derivatives, their preparation and medicinal compositions containing them

(30) Priorité: 24.02.1983 FR 8303004
(43) Date de publication de la demande: 26.09.1984
(73) Titulaire: RHONE-POULENC SANTE, 92400 Courbevoie Cédex (FR)
(72) Inventeur: Bourzat, Jean-Dominique, F-75015 Paris (FR); Cotrel, Claude, F-75012 Paris (FR); Farge, Daniel, F-94320 Thiais (FR); Paris, Jean-Marc, F-77360 Vaires s/Marne (FR); Taurand, Gérard, F-94000 Créteil (FR)
(74) Mandataire: Le Goff, Yves

## Description

La présente invention concerne de nouveaux dérivés de la dithiole-1,2 one-3 de formule générale: 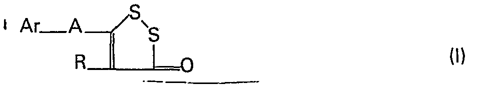 dans laquelle
- soit R représente un atome d'hydrogène ou de chlore, A représente un atome d'oxygène ou un radical imino, alcoylimino, alcoylcarbonylimino ou formylimino et Ar représente un radical phényle substitué par un radical cycloalcoyle dont la partie alcoyle contient 3 à 7 atomes de carbone, alcényle contenant 2 à 4 atomes de carbone en chaîne droite ou ramifiée, anilino, formyle, semicarbazonométhyle, dithiolanne-1,3 yle-2, dioxolanne-1,3 yle-2, dialcoylaminométhylèneamino, trifluorométhylthio, alcoylcarbonyloxy ou alcoyle substitué par un radical hydroxy, alcoyloxy, carboxy, amino, alcoylcarbonylamino, alcoyloxycarbonylamino, dialcoylaminométhylèneamino, formamido, alcoyluréido, phénylsulfonyle, carba- moyle, alcoylcarbamoyle, dialcoylcarbamoyle, formyle, semicarbazonométhyle, dithiolanne-1,3 yle-2 ou dioxolanne-1,3 yle-2 ou bien Ar représente un radical pyridyle, quinolyle, isoquinolyle, indanyle ou quinoxalinyle,
- soit R représente un atome d'hydrogène, A représente un atome d'oxygène, ou un radical imino, alcoylimino, alcoylcarbonylimino ou formylimino et Ar représente un radical phényle substitué par un radical alcoyle,
- soit R représente un atome de chlore, A représente un atome d'oxygène ou un radical alcoylcarbonylimino ou formylimino et Ar représente un radical phényle substitué par un radical alcoyle, étant entendu que tous les radicaux alcoyle et portions alcoyle cités ci-dessus ou qui seront cités par la suite contiennent, sauf mention spéciale, à à 4 atomes de carbone en chaîne droite ou ramifiée, ainsi que, lorsqu'ils existent, leurs sels d'addition avec les acides, leurs sels métalliques et leurs sels d'addition avec les bases organiques.

Selon l'invention, les produits de formule générale (1) dans laquelle A représente un atome d'oxygène ou un radical imino ou alcoylimino, R représente un atome de chlore et Ar est défini comme précédemment à l'exception de représenter un radical phényle substitué par un radical dialcoylaminométhylèneamino ou un radical alcoyle substitué par un radical dialcoylaminométhylèneamino, peuvent être préparés par action d'un produit de formule générale: 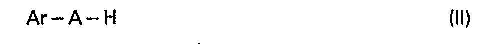 dans laquelle Ar et A ont les définitions correspondantes sur la dichloro-4,5 dithiole-1,2 one-3 de formule:

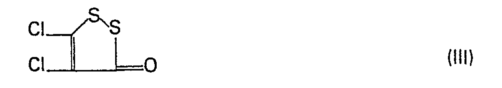

On opère généralement dans un solvant organique tel qu'un alcool comme le méthanol, une cétone comme l'acétone, ou le diméthylformamide (de préférence le diméthylformamide lorsque A représente un atome d'oxygène), en présence d'un léger excès par rapport à la quantité théorique d'un accepteur d'acide tel qu'un carbonate ou bicarbonate alcalin comme le carbonate ou le bicarbonate de potassium, ou encore une base organique comme le nitrilo-tris (propanol-2), à une température comprise entre 0 et 80 °C, de préférence voisine de 20 °C.

La dichloro-4,5 dithiole-1,2 one-3 peut être préparée selon la méthode décrite par F. Boberg, Ann. Chem. 681, 169 (1965).

Les produits de formule générale (II) peuvent être obtenus par application ou adaptation des méthodes connues à la portée de l'homme du métier.

Selon l'invention, les produits de formule générale (I) dans laquelle A représente un radical alcoylimino et les autres symboles sont définis comme précédemment peuvent être préparés par action d'un produit de formule générale: 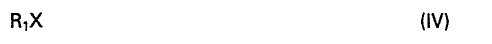 dans laquelle R₁ représente un radical alcoyle et X représente un atome d'halogène ou un rester d'ester réactif tel que mésyloxy ou tosyloxy, sur un produit de formule générale (I) dans laquelle A représente un radical imino et les autres symboles sont définis comme précédemment, c'est-à-dire un produit de formule générale:

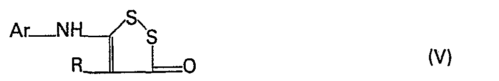

La condensation du produit de formule générale (IV) sur le produit de formule générale (V) s'effectue en général dans un solvant organique tel que l'acétonitrile en présence d'un léger excès par rapport à la quantité théorique d'un agent alcalin de condensation tel qu'un alcoolate alcalin comme le méthylate de sodium ou un hydrure alcalin tel que l'hydrure de sodium, à une température comprise entre 15 et 60 °C.

Selon l'invention, les produits de formule générale (I) dans laquelle A représente un radical alcoylcarbonylimino ou formylimino et les autres symboles sont définis comme précédemment peuvent être préparés par action d'un anhydride mixte de formule générale: 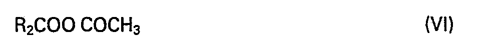 dans laquelle R₂ représente un atome d'hydrogène ou un radical alcoyle, sur un produit de formule générale (V).

La réaction s'effectue généralement dans un solvant organique tel que la pyridine à une température comprise entre 0 et 20 °C.

Selon l'invention, les produits de formule générale (I) dans laquelle R représente un atome d'hydrogène et les autres symboles sont définis comme précédemment peuvent être préparés par déchloration d'un produit de formule générale (I) dans laquelle R représente un atome de chlore et les autres symboles sont définis comme précédemment, c'est-à-dire un produit de formule générale:

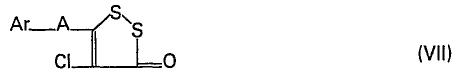

La déchloration peut être effectuée par toute méthode connue de l'homme du métier pour éliminer un atome de chlore sans toucher au reste de la molécule. Il est particulièrement avantageux d'effectuer la réaction au moyen d'au moins une mole d'un hydrure de trialcoylétain comme l'hydrure de tributylétain à une température voisine de 20 °C.

Selon l'invention, les produits de formule générale (I) dans laquelle Ar représente un radical phényle substitué par un radical dithiolanne-1,3 yle-2, dioxolanne-1,3 yl-2 ou alcoyle substitué par un radical dithiolanne-1,3 yl-2 ou dioxolanne-1,3 yle-2 et les autres symboles sont définis comme précédemment peuvent être préparés par action d'un produit de formule générale: 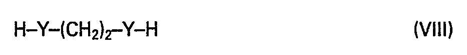 dans laquelle les symboles Y représentent tous deux un atome d'oxygène ou un atome de soufre, sur un produit de formule générale (I) dans laquelle Ar représente un radical phényle substitué par un radical formyle ou alcoyle substitué par un radical formyle, c'est-à-dire un produit de formule générale: 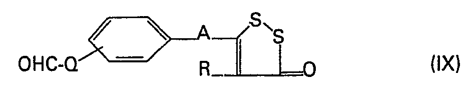 dans laquelle Q représente une liaison de valence ou un radical alcoylène contenant 1 à 4 atomes de carbone en chaîne droite ou ramifiée, et les autres symboles sont définis comme précédemment.

La réaction s'effectue généralement dans un solvant chloré tel que le chloroforme en présence d'une quantité catalytique d'acide p-toluènesulfo- nique à une température comprise entre 20 °C et le reflux du mélange réactionnel.

Selon l'invention, les produits de formule générale (I) dans laquelle Ar représente un radical phényle substitué par un radical dialcoylaminométhylèneamino ou alcoyle substitué par un radical dialcoylaminométhylèneamino et les autres symboles sont définis comme précédemment peuvent être préparés par action d'un produit de formule générale: 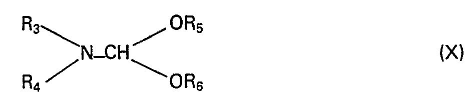 dans laquelle les radicaux R₃, R₄, R₅ et R₆ représentent des radicaux alcoyle, sur un produit de formule générale (I) dans laquelle Ar représente un radical phényle substitué par un radical amino ou alcoyle substitué par un radical amino, c'est-à-dire un produit de formule générale: 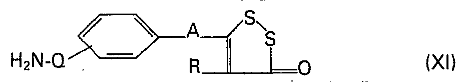 dans laquelle Q représente une liaison de valence ou un radical alcoylène contenant 1 à 4 atomes de carbone en chaîne droite ou ramifiée et les autres symboles sont définis comme précédemment.

La réaction s'effectue généralement sans solvant ou dans un solvant organique tel qu'un alcool (éthanol) un solvant chloré (chlorure de méthylène) ou le diméthylformamide à une température comprise entre 10 et 80 °C.

Selon l'invention, les produits de formule générale (I) dans laquelle Ar représente un radical phényle substitué par un radical alcoyle substitué par un radical alcoylcarbonylamino ou alcoyloxycarbonylamino et les autres symboles sont définis comme précédemment peuvent être préparés par action d'un produit de formule générale: 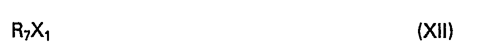 dans laquelle R₇ représente un radical alcoylcar- bonyle ou alcoyloxycarbonyle et X, représente un atome d'halogène, sur un produit de formule générale (I) dans laquelle Ar représente un radical phényle substitué par un radical alcoyle substitué par un radical amino, et les autres symboles sont définis comme précédemment, c'est-à-dire un produit de formule générale: 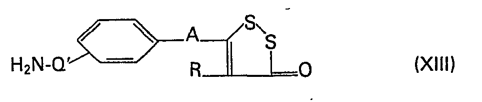 dans laquelle Q' représente un radical alcoylène contenant 1 à 4 atomes de carbone en chaîne droite ou ramifiée et les autres symboles sont définis comme précédemment.

La réaction s'effectue généralement dans un solvant tel que la pyridine à une température comprise entre -20 et 80 °C.

Selon l'invention, les produits de formule générale (I) dans laquelle Ar représente un radical phényle substitué par un radical alcoyle substitué par un radical formamido et les autres symboles sont définis comme précédemment peuvent être préparés par action d'un formiate d'alcoyle de formule générale: 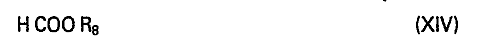 dans laquelle Rε représente un radical alcoyle, sur un produit de formule générale (XIII).

La réaction s'effectue généralement dans un excès de formiate d'alcoyle de formule générale (XIV) à une température comprise entre 0 et 60 °C.

Selon l'invention, les produits de formule générale (1) dans laquelle Ar représente un radical phényle substitué par un radical alcoyle substitué par un radical uréido et les autres symboles sont définis comme précédemment peuvent être préparés par action d'un isocyanate de formule générale: 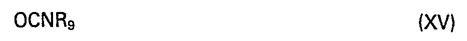 dans laquelle R₉ représente un radical alcoyle, sur un produit de formule générale (XIII).

La réaction s'effectue généralement dans un solvant organique tel qu'un solvant chloré comme le chlorure de méthylène à une température comprise entre 0 et 60 °C.

Les nouveaux produits de formule générale (I) peuvent être purifiés par les méthodes habituelles telles que cristallisation, chromatographie ou, le cas échéant, extractions successives en milieu acide ou basique.

Les nouveaux produits de formule générale (I) dans laquelle le substituant Ar est porteur d'une fonction aminée tel qu'il est défini précédemment peuvent être éventuellement transformés en sels d'addition avec les acides par action d'un acide dans un solvant organique tel qu'un alcool, une cétone, un ester ou un solvant chloré. Le sel précipite, éventuellement après concentration de sa solution; il est séparé par filtration ou décantation.

De manière analogue, les produits de formule générale (I) dans laquelle Ar est porteur d'une fonction acide tel qu'il est défini précédemment peuvent être éventuellement transformés en sels métalliques ou en sels d'addition avec les bases organiques.

Il est entendu pour l'homme du métier que, pour la mise en oeuvre des procédés selon l'invention décrits précédemment, il peut être nécessaire d'introduire des groupements protecteurs des fonctions présentes dans le radical Ar afin d'éviter les réactions secondaires avant de mettre en oeuvre l'un ou l'autre des procédés selon l'invention. Le groupement protecteur pourra être ensuite conservé ou éliminé selon que le produit obtenu est ou non un produit selon l'invention. Ainsi, lorsque le radical Ar est porteur d'une fonction aldéhyde, il est avantageux de protéger cette fonction par exemple sous forme de semicarbazone et d'éliminer ensuite, si on le désire, le groupement protecteur en milieu alcoolique en présence de sulfate de cuivre. De même, lorsque Ar est porteur d'une fonction amino ou alcoylamino, il est nécessaire de protéger ces fonctions par exemple par un radical tert-butyloxycarbonyle, puis de libérer la fonction amino en milieu acide aqueux tel qu'une solution aqueuse d'acide chlorhydrique ou mieux une solution acétique de gaz chlorhydrique. Lorsque Ar représente un radical porteur d'une fonction acide, il est avantageux de protéger cette fonction par exemple sous forme d'un ester tel qu'un ester éthylique ou tert-butylique puis de libérer, si on le désire, la fonction acide par hydrolyse en milieu acide par exemple une solution aqueuse d'acide chlorhydrique ou mieux une solution acétique de gaz chlorhydrique.

On connaît déjà par les brevets allemands 1242324 et 1 278701 et par le certificat d'addition n° 94485 au brevet français 1498374 des amino-5-chloro 4-dithiole-1,2 one-3; aucun de ces documents ne divulgue les produits selon l'invention ni ne suggère une application thérapeutique pour ce type de produits.

Les nouveaux produits selon l'invention et leurs sels, lorsqu'ils existent, présentent d'intéressantes propriétés pharmacologiques les rendant susceptibles d'application dans le traitement de fond de la maladie rhumatismale. Ils se sont montrés actifs à des concentrations voisines de 5.10-⁶M dans le test de la mesure de l'activation des macrophages in vitro, effectué selon la technique de J. Schnyder et M. Baggiolini (J. Exp. Med.: 148, 1449,1978).

En outre, les produits de formule générale (I) présentent une faible toxicité. Leur DL₅₀ est généralement supérieure à 900 mg/kg par voie orale chez la souris en administration unique.

Pour l'emploi médicinal, il peut être fait usage des produits selon l'invention tels quels ou, lorsqu'ils existent, à l'état de sels pharmaceutiquement acceptables, c'est-à-dire non toxiques aux doses d'utilisation.

Comme sels pharmaceutiquement acceptables, on peut citer les sels d'addition avec les acides minéraux tels que chlorhydrates, sulfates, nitrates, phosphates ou organiques tels que acétates, propionates, succinates, benzoates, fuma- rates, théophilline-acétates, salicylates, phénol- phtalinates, méthylène-bis-β-oxynaphtoates ou des dérivés de substitution de ces composés. Comme sels pharmaceutiquement acceptables, on peut encore citer les sels avec les métaux alcalins tels que le sodium, le potassium ou le lithium, avec les métaux alcalinoterreux tels que les sels de calcium et de magnésium, et les sels d'addition avec les bases organiques tels que les sels d'éthanolamine et de lysines.

Les exemples suivants, donnés à titre non limitatif, montrent comment l'invention peut être mise en pratique.

### Exemple 1

A une suspension de 3,74 g de dichloro-4,5 dithiole-1,2 one-3 et de 2,2 g de bicarbonate de potassium dans 30 cm³ de méthanol, on ajoute 2,92 g de cyclopropyl-4-aniline. Après 20 h d'agitation à une température voisine de 20 °C, on coule 60 cm³ d'eau distillée dans le mélange réactionnel; le produit insoluble est séparé par filtration et lavé 3 fois par 30 cm³ au total d'eau distillée. Par recristallisation du produit ainsi obtenu dans 75 cm³ d'acétate d'éthyle, on obtient 3,8 g de chloro-4 (cyclopropyl-4-phénylamino)-5^{>} dithiole-1,2 one-3 fondant à 154 °C.

### Exemple 2

A une suspension de 7 g de dichloro-4,5 dithiole-1,2 one-3 et de 3,7 g de bicarbonate de potassium dans 80 cm³ de méthanol on ajoute 5,5g de cyclobutyl-4 aniline. Après 20 h d'agitation à une température voisine de 20 °C, on coule 80 cm³ d'eau distillé dans le mélange réactionnel; l'émulsion obtenue est extraite 2 fois par 160 cm³ au total de chlorure de méthylène. Les phases organiques sont réunies, séchées sur sulfate de sodium, filtrées et concentrées à sec sous pression réduite (2,7 kPa) à 40 °C. Le produit obtenu est ensuite dissous dans 50 cm³ de chlorure de méthylène et la solution obtenue est versée sur 300 g de gel d'alumine contenus dans une colonne de 4,5 cm de diamètre. On élue d'abord par 300 cm³ de chlorure de méthylène; l'éluat correspondant est éliminé. On élue ensuite par 500 cm³ de chlorure de méthylène; l'éluat correspondant est concentré à sec sous pression réduite (2,7 kPa) à 40 °C. Par recristallisation du résidu ainsi obtenu dans 18cm³ d'acétonitrile, on obtient 3,6 g de chloro-4 (cyclobutyl-4 phényl amino)-5 dithiole-1,2 one-3 fondant à 100 °C.

La cyclobutyl-4 aniline peut être préparée par hydrogénolyse de 14 g (hydroxy-1 cyclobutyl)-4 aniline en solution dans 70 cm³ d'éthanol en présence de 4,3 g de palladium à 10% sur noir de carbone sous une pression de 310 kPa à une température voisine de 20 °C pendant 20 h. Après filtration du catalyseur, le mélange réactionnel est concentré à sec sous pression réduite (2,7 kPa) à 40 °C; le produit obtenu est ensuite dissous dans 20 cm³ de chlorure de méthylène et la solution obtenue est versée sur 300 g de gel d'alumine contenus dans une colonne de 3,4 cm de diamètre. On élue par 200 cm³ d'un mélange chlorure de méthylène et de cyclohexane (70/30 en volumes); l'éfluat correspondant est éliminé. On élue ensuite par 500 cm³ du même mélange; l'éluat correspondant est concentré à sec sous pression réduite (2,7 kPa) à 40 °C. On obtient 5,5 g de cyclobutyl-4 aniline sous forme d'huile brune [(Rf = 0,5, chromatographie sur couche mince de gel d'alumine, solvant: chlorure de méthylène/cyclohexane (70/ 50 en volumes)].

L'(hydroxy-1 cyclobutyl)-4 aniline peut être préparée de la façon suivante: 31,6 g de bromo-4 N,N-bis (triméthylsilyl) aniline dissous dans 100 cm³ d'éther éthylique sont traités pendant 24 h à une température voisine de 20 °C par 62 cm³ d'une solution 1,6 M de butyllithium dans l'hexane. A la solution obtenue, on ajoute à-10 °C une solution de 7 g de cyclobutanone dans 70 cm³ d'éther éthylique, puis on agite 2 heures à une température voisine de 20 °C. Le mélange réactionnel est ensuite hydrolysé par 210 cm³ d'une solution aqueuse à 10% de chlorure d'ammonium. La phase organique est extraite par 100 cm³ d'une solution aqueuse d'acide chlorhydrique 2 N; la phase aqueuse est lavée 2 fois par 100 cm³ au total d'éther éthylique puis alcalinisée à pH 8 par addition de soude aqueuse 4 N. L'émulsion obtenue est extraite par 100 cm³ d'éther éthylique; la phase organique est lavée 2 fois par 200 cm³ au total d'eau distillée, séchée sur sulfate de sodium, filtrée et concentrée à sec sous pression réduite (2,7 kPa) à 40 °C. On obtient 14 g d'(hydroxy-1 cyclobutyl)-4 aniline sous forme d'huile brune. [Rf = 0,36; chromatographie sur couche mince de gel de silice; solvant: chlorure de méthylène/métha- nol (95/5 en volumes)].

### Exemple 3

En opérant de manière analogue à celle décrite à l'exemple 1 mais à partir de 11,2 g de dichloro-4,5 dithiole-1,2 one-3 et de 10,6 g de cyclopentyl-4-aniline, on obtient, après recristallisation dans l'éthanol, 14,8 g de chloro-4 (cyclopentyl-4 phénylamino)-5 dithiole-1,2 one-3 fondant à 119 °C.

La cyclopentyl-4 aniline peut être préparée selon P.V. Hai, N.P. Buu-Hoi et N.D. Xuong J. Org. Chem. 23, 39 (1958).

### Exemple 4

En opérant de manière analogue à celle décrit à l'exemple 2 mais à partir de 5,6 g de dichloro-4,5 dithiole-1,2 one-3 et de 4,9 g de cyclopentyl-3 aniline, on obtient 9,8 g de produit brut huileux. Ce produit est dissous dans 50 cm³ de chlorure de méthylène et la solution obtenue est versée sur 200 g de gel de silice contenus dans une colonne de 3,8 cm de diamètre. On élue d'abord par 600 cm³ de chlorure de méthylène et l'éluat correspondant est éliminé. On élue ensuite avec 1800cm³ de chlorure de méthylène et l'éluat correspondant est concentré à sec sous pression réduite (2,7 kPa) à 40 °C. Par recristallisation du produit ainsi obtenu dans un mélange d'acétate d'éthyle et d'oxyde d'isopropyle (½ en volumes), on obtient 4 g de chloro-4 (cyclopentyl-3 phénylamino)-5 dithiole-1,2 one-3 fondant à 74 °C.

La cyclopentyl-3 aniline peut être préparée de manière analogue à celle décrite à l'exemple 2 pour la préparation de la cyclobutyl-4 aniline.

A partir de 34 g de bromo-3 N,N-bis (triméthylsilyl) aniline et de 9,1 g de cyclopentanone, on obtient 5 g de cyclopentyl-3 aniline sous forme d'huile jaune utilisée telle quelle dans la synthèse décrite précédemment.

### Exemple 5

En opérant de manière analogue à celle décrite à l'exemple 1, mais à partir de 9,4 g de dichloro-4,5 dithiole-1,2 one-3 et de 8,8 g de cyclohexyl-4 aniline, on obtient après recristallisation dans l'acétonitrile 11 g de chloro-4 (cyclohexyl-4 phénylamino)-5 dithiole-1,2 one-3 fondant à 160 °C.

### Exemple 6

En opérant de manière analogue à celle décrite à l'exemple 1, mais à partir de 7,4 g de dichloro-4,5 dithiole-1,2 one-3 et de 7,5 g de cycloheptyl-4 aniline, on obtient après recristallisation dans l'acétonitrile 7,4 g de chloro-4 (cycloheptyl-4 phénylamino)-5 dithiole-1,2 one-3 fondant à 160 °C.

La cycloheptyl-4 aniline peut être préparée de manière analogue à celle décrite à l'exemple 2 pour la préparation de la cyclobutyl-4 aniline. A partir de 22,2 g de bromo-4 N,N-bis (triméthylsilyl) aniline et de 7,9 g de cycloheptanone, on obtient 9,7 g de cycloheptyl-4 aniline sous forme d'huile brune utilisée telle quelle dans la synthèse décrite précédemment.

### Exemple 7

A une suspension de 15,6 g de chloro-4 (cyclopentyl-4 phénylamino)-5 dithiole-1,2 one-3, préparée comme à l'exemple 3, dans 200 cm³ d'acétoni- triole, on ajoute 4,1 g de méthylate de sodium puis on agite la suspension obtenue pendant 1 h à une température voisine de 20 °C. On ajoute ensuite 12,1 g d'iodure de méthyle et agite pendant 20 h à une température voisine de 20 °C.

Le mélange réactionnel est ensuite versé lentement dans 2000 cm³ d'eau distillée puis extrait 1 fois par 500 cm³ puis 2 fois par 500 cm³ au total d'éther éthylique. Les phases organiques sont réunies et séchées sur sulfate de sodium, filtrées puis concentrées à sec sous pression réduite (2,7 kPa) à 60 °C. Le résidu huileux obtenu est dissous dans 30 cm³ de chlorure de méthylène et la solution obtenue est versée sur 330 g de gel de silice contenus dans une colonne de 4,5 cm de diamètre. On élue d'abord par 500 cm³ de chlorure de méthylène; l'éluat correspondant est éliminé. On élue ensuite par 700 cm³ de chlorure de méthylène; l'éluat correspondant est concentré à sec sous pression réduite (2,7 kPa) à 40 °C. Après recristallisation du produit obtenu dans 50 cm³ de cyclohexanne, on obtient 4,9 g de chloro-4 (N-méthyl cyclopentyl-4 phénylamino)-5 dithiole-1,2 one-3 fondant à 73 °C.

### Exemple 8

En opérant de manière analogue à celle décrite à l'exemple 1, mais à partir de 9,4 g de dichloro-4,5 dithiole-1,2 one-3 et de 10,8 g d'(amino-4 phényl)-2 dithiolanne-1,3, on obtient, après recristallisation dans un mélange de diméthylformamide et d'eau (90/10 en volumes), 10,1 g de chloro-4 [(dithiolanne-1,3 yl-2)-4 phénylamino]-5 dithiole-1,2 one-3 fondant à 198 °C.

L'(amino-4 phényl)-2 dithiolanne-1,3 peut être obtenu de la manière suivante:
A une suspension de 22,7-g de (nitro-4 phényl)-2 dithiolanne-1,3 et de 42 g de fer en poudre dans 250 cm³ d'éthanol à 20% d'eau, on ajoute, en agitant à 60 °C, 3 cm³ d'acide chlorhydrique concentré (d = ₁,₁₉). Le mélange réactionnel est chauffé à reflux pendant 30 min. Le mélange réactionnel est ensuite filtré à chaud et le filtrat est concentré à sec sous pression réduite (2,7 kPa) à 50 °C. Le résidu obtenu est dissous dans 250 cm³ de chlorure de méthylène et la solution obtenue est lavée par 100 cm³ d'une solution aqueuse de soude 0,3 N puis 2 fois par:100 cm³ au total d'eau distillée; elle--est ensuite séchée sur sulfate de magnésium, filtrée et concentrée à sec sous pression réduite (2,7 kPa) à 80 °C. On obtient ainsi 18g d'(amino-4 phényl)-2 dithiolanne-1,3 fondant à 69°C.

Le .(nitro-4-phényl)-2 dithiolanne-1,3 peut être préparé en chauffant à reflux une suspension de 75,5 g de nitro-4 benzaldéhyde dans 500 cm³ de méthylcyclohexane contenant 65,8 g d'éthanedi- thiole-1,2 et cm³ d'éthérate de trifluorure de bore pendant 3,5 h tout en éliminant l'eau formée par distillation azéotropique. La solution obtenue est ensuite refroidie à une température voisine de 20°C et le produit insoluble est séparé par filtration, lavé 2 fois avec 100 cm³ de méthylcyclohexane au total puis dissous dans 500 cm³ de chlorure de méthylène. La solution obtenue est lavée 2 fois par 500 cm³ au total de solution aqueuse de soude 1 N, puis 3 fois par 600 cm³ au total d'eau distillée, elle est ensuite séchée sur sulfate de magnésium et concentrée à sec sous pression réduite (2,7 kPa) à 80 °C. On obtient ainsi 112 g de (nitro-4-phényl)-2 dithiolanne-1,3 fondant à 78 °C.

### Exemple 9

En opérant de manière analogue à celle décrite à l'exemple 1, mais à partir de 66,1 g de dichloro-4,5 dithiole-1,2 one-3 et de 69,2 g de semicarbazone d'amino-4-benzaldéhyde, on obtient, après recristallisation dans le méthanol, 53,1 g de chloro-4 (semicarbazonométhyl-4 phénylamino)-5 di- thiolo-1,2 one-3 fondant à 193 °C.

La semicarbazone de l'amino-4 benzaldéhyde peut être préparée par hydrogénation catalytique de 104 g de semicarbazone de nitro-4 benzaldéhyde dans l'éthanol en présence de palladium à 3% sur noir de carbone à une température voisine de 25 °C sous une pression voisine de 100 kPa pendant 2 h. Après séparation du catalyseur par filtration, le mélange réactionnel est concentré à sec sous pression réduite (2,7 kPa) à 60 °C. Le résidu ainsi obtenu est lavé par 100 cm³ d'éthanol; après séchage sous pression réduite (2,7 kPa), on obtient 69,3 g de semicarbazone d'amino-4 benzaldéhyde fondant à 124 °C.

### Exemple 10

On opère comme à l'exemple 9 puis on libère la fonction aldéhyde de son groupement protecteur de la façon suivante:
Une solution de 36,4 g de chloro-4 (semicarbazonométhyl-4 phénylamino)-5 dithiole-1,2 one-3 et de 138,2 g de sulfate de cuivre pentahydraté dans un mélange de 1850 cm³ de méthanol, de 1850 cm³ de tétrahydrofuranne et de 3700 cm³ d'eau distillée est chauffée au reflux pendant 5 h. La suspension obtenue est versée dans 5000 cm³ d'acétate d'éthyle et la phase aqueuse est décantée; la phase organique est lavée par 3000 cm³ d'eau distillée au total, séchée sur du sulfate de magnésium, filtrée puis concentrée à sec sous pression réduite (2,7 kPa) à 50 °C. On obtient ainsi 22,8 g de résidu qui est ensuite absorbé sur 100 g de gel de silice; le mélange obtenu est déposé sur 600 g de gel de silice contenus dans une colonne de 5,5 cm de diamètre. On élue d'abord par 6000cm³ de chlorure de méthylène et l'éluat correspondant est éliminé; on élue ensuite par 7000 cm³ de chlorure de méthylène et l'éluat correspondant est concentré à sec sous pression réduite (2,7 kPa) à 40 °C. Par recristallisation du résidu obtenu dans l'acétonitrile, on obtient 6,5 g de chloro-4 (formyl-4 phénylamino)-5 dithiole-1,2 one-3 fondant à 218°C avec décomposition.

### Exemple 11

Une suspension de 5,7 g de chloro-4 (formyl-4 phénylamino)-5 dithiole-1,2 one-3 dans 600 cm³ de chloroforme contenant 0,2 g d'acide parato- luène sulfonique et 1,43 g d'éthylène glycol est chauffée au reflux pendant 3 h, l'eau formée étant éliminée par distillation azéotropique. Après refroidissement à une température voisine de 20 °C, le mélange réactionnel est lavé par 100 cm³ d'eau distillée puis la phase organique est séchée sur sulfate de magnésium, filtrée et concentrée à sec sous pression réduite (2,7 kPa) à 40 °C. Après recristallisation du résidu obtenu dans le méthanol, on obtient 2,8 g de chloro-4 [(dioxolanne-1,3 yl-2)-4 phénylamino]-5 dithiole-1,2 one-3 fondant à 168 °C.

### Exemple 12

En opérant de manière analogue à celle décrite à l'exemple 1, mais à partir de 4,88 g de dichloro-4,5 dithiole-1,2 one-3 et de 4,8 g d'amino-4 diphénylamine, on obtient, après recristallisation dans l'acétonitrile, 4,9 g de chloro-4 (phénylamino-4 phénylamino)-5 dithiole-1,2 one-3 fondant à 186°C.

L'amino-4 diphénylamine peut être préparée par hydrogénation catalytique de 6,8 g de nitro-4 diphénylamine dans l'éthanol en présence de 0,3g de palladium à 10% sur noir de carbone à une température voisine de 25 °C sous une pression voisine de 100 kPa pendant 1 h. Après séparation du catalyseur par filtration, le mélange réactionnel est concentré à sec sous pression réduite (2,7 kPa) à 50 °C. On obtient ainsi 5,8 g d'amino-4 diphénylamine sous forme d'huile violacée. [Rf = 0,50, chromatographie sur couche mince de gel de silice; éluant: chlorure de méthylène/acétate d'éthyle (90/10 en volumes)].

### Exemple 13

En opérant de manière analogue à celle décrite à l'exemple 1, mais à partir de 30 g de dichloro-4,5 dithiole-1,2 one-3 et de 21 g d'amino-4 styrène, on obtient, après recristallisation dans l'acétonitrile, 6,4 g de chloro-4 (vinyl-4 phénylamino)-5-dithiole-1,2 one-3 fondant à 185 °C.

L'amino-4 styrène peut être préparé par la méthode décrite par A.M. Shur et N.A. Barba Chem. Abstr. 64, P 3383 h.

### Exemple 14

En opérant comme à l'exemple 13 mais en remplaçant l'amino-4 styrène par la (propène-2 yl)-4 aniline, on obtient, après recristallisation dans l'oxyde d'isopropyle, la chloro-4 [(propène-2 yl)-4 phénylamino]-5 dithiole-1,2 one-3 fondant à 129°C.

### Exemple 15

En opérant de manière analogue à celle décrite à l'exemple 2, mais à partir de 65 g de dichloro-4,5 dithiole-1,2 one-3 et de 57,6 g d'(hydroxy-1 méthyl-1 éthyl)-4 aniline, on obtient 117 g de produit brut sous forme d'huile rouge. Ce produit est dissous dans 250 cm³ de chlorure de méthylène et la solution obtenu est versée sur 2500 g de gel de silice contenus dans une colonne de 9 cm de diamètre. On élue d'abord par 16 I de chlorure de méthylène et d'acétate d'éthyle (85/15 en volumes); les éluats correspondants sont éliminés. On élue ensuite par 5 I d'un mélange de chlorure de méthylène et d'acétate d'éthyle (70/30 en volumes); l'éluat correspondant est concentré à sec sous pression réduite (2,7 kPa) à 40 °C. On obtient, après recristallisation dans l'acétonitrile, 5,1 g de chloro-4 [(hydroxy-1 méthyl-1 éthyl)-4 phénylamino]-5 dithiole-1,2 one-3 fondant à 162 °C.

L'(hydroxy-1 méthyl-1 éthyl)-4 aniline peut être préparée en ajoutant une solution de 54 g d'amino-4 acétophénone dans 1000 cm³ de tétrahydrofuranne à 1000 cm³ d'une solution 1,2 M d'iodure de méthyl magnésium dans le tétrahydrofuranne maintenue à une température voisine de 5 °C. La suspension obtenue est agitée ensuite une nuit à une température voisine de 20 °C, puis elle est versée dans 5000 cm³ de solution aqueuse saturée de chlorure d'ammonium. La phase organique est séparée par décantation, la phase aqueuse est extraite 3 fois par 3000 cm³ au total d'éther éthylique. Les phases organiques sont réunies est séchées sur sulfate de magnésium, filtrées, puis concentrées à sec sous pression réduite (2,7 kPa) à 40 °C. On obtient ainsi 57,6 g d'(hydroxy-1 méthyl-1 éthyl)-4 aniline sous forme d'huile brune [Rf = 0,37; chromatographie sur couche mince de gel de silice; éluant chlorure de méthylène/acétate d'éthyle (50/50 en volumes)]..

### Exemple 16

En opérant d'une manière analogue à celle décrite à l'exemple 1, mais à partir de 13,1 g de dichloro-4,5 dithiole-1,2 one-3 et de 9,6 g d'alcool amino-4 benzylique, on obtient, après recristallisation dans l'acétonitrile, 4,5 g de chloro-4 (hy- droxyméthyl-4 phénylamino)-5 dithiole-1,2 one-3 fondant à 168°C.

### Exemple 17

A une suspension de 6 g de bicarbonate de potassium et de 10,1 g de dichloro-4,5 dithiole-1,2 one-3 dans 75 cm³ de méthanol, on ajoute 8,2g d'(amino-4 phényl)-2 éthanol. On agite ensuite le mélange réactionnel à une température voisine de 20 °C pendant 20 h puis on ajoute 75 cm³ d'eau distillée. L'émulsion obtenue est extraite par 75 cm³ de chlorure de méthylène et la phase organique est ensuite lavée par 20 cm³ d'eau distillée, séchée sur sulfate de magnésium, filtrée puis concentrée à sec sous pression réduite (2,7 kPa) à 40°C. Après recristallisation du produit ainsi obtenu dans un mélange d'acétate d'éthyle et d'oxyde d'isopropyle (50/50 en volumes), on obtient 5,3 g de chloro-4 [(hydroxy-2 éthyl)-4 phénylamino]-5 dithiole-1,2 one-3 fondant à 99 °C.

L'(amino-4 phényl)-2 éthanol peut être préparé par hydrogénation catalytique de 10 g de (nitro-4 phényl)-2 éthanol dans 300 cm³ d'éthanol en présence de 0,9 g de palladium à 3% sur noir de carbone sous une pression voisine de 100 kPa pendant 14 h à une température voisine de 20 °C. Après séparation du catalyseur par filtration, le mélange réactionnel est concentré à sec sous pression réduite (2,7 kPa) à 40 °C. On obtient ainsi 8,2 g d'(amino-4 phényl)-2 éthanol fondant à 109°C.

### Exemple 18

En opérant de manière analogue à celle décrite à l'exemple 2, mais à partir de 10 g de dichloro-4,5 dithiole-1,2 one-3 et de 11,35 g de trifluorométhylthio-3 aniline, on obtient, après recristallisation dans un mélange d'oxyde d'isopropyle et d'acétate d'éthyle (60/40 en volumes), 3,6 g de chloro-4 (trifluorométhylthio-3 phénylamino)-5 dithiole-1,2 one-3 fondant à 160°C.

### Exemple 19

En opérant de manière analogue à celle décrite à l'exemple 2, mais à partir de 7,5 g de dichloro-4,5 dithiole-1,2 one-3 et de 10,9 g d'(amino-4 benzyl)-phénylsulfone, on obtient, après recristallisation dans l'acétonitrile, 2,2 g de chloro-4 [(phényl- sulfonylméthyl-4) phénylamino]-5 dithiole-1,2 one-3 fondant à 186 °C.

L'(amino-4 benzyl) phényl sulfone peut être préparée selon la méthode décrite par W.R. Waldron et E.E. Reid J. Am. Chem. Soc. 45, 2399 (1923).

### Exemple 20

Une solution de 20,5 g de [(chloro-4 oxo-3 dithiole-1,2 yl-5) amino-4 phényl]-3 butyrate d'éthyle dans 360 cm³ d'acide acétique contenant 57,5 cm³ d'une solution aqueuse d'acide sulfurique 18 N est chauffée au reflux pendant 30 min. Le mélange réactionnel est refroidi à une température voisine de 20 °C et versé dans 1200 cm³ d'eau distillée. Le produit insoluble est séparé par filtration et séché à l'air à une température voisine de 20 °C. Après recristallisation dans l'éthanol, on obtient 10,1 g d'acide [(chloro-4 oxo-3 dithiole-1,2 yl-5) amino-4 phényl]-3 butyrique fondant à 210°C. Le [(chloro-4 oxo-3 dithiole-1,2 yl-5) amino-4 phényl]-3-butyrate d'éthyle peut être préparé en opérant de manière analogue à celle décrite à l'exemple 1 mais à partir de..18,7 g de dichloro-4,5 dithiole-1,2 one-3 et de 22,8 g d'(amino-4 phényl)-3 butyrate d'éthyle; on obtient ainsi 31,1 g de [(chloro-4 oxo-3.dithiole-1,2 yl-5) amino-4 phényl]-3 butyrate d'éthyle fondant à 98 °C.

L'(amino-4 phényl)-3 butyrate d'éthyle peut être préparé par hydrogénation catalytique de 95,8 g de (nitro-4 phényl)-3 butène-2-oate d'éthyle dans 3200 cm³ d'éthanol en présence de 3,7 g de palladium à 10% sur noir de carbone, sous une pression voisine de 100 kPa, à une température voisine de 25 °C pendant 45 min. Après séparation du catalyseur par filtration, le mélange réactionnel est concentré à sec sous pression réduite (2,7 kPa) à 50 °C; on obtient ainsi 83,4 g d'(amino-4 phényl)-3 butyrate d'éthyle sous forme d'huile brune [Rf = 0,47; chromatographie sur couche mince de gel de silice; éluant: chlorure de méthylène/acétate d'éthyle 90/10 en volumes].

Le (nitro-4 phényl)-3 butène-2-oate d'éthyle peut être préparé en ajoutant goutte à goutte 134,4-g de diéthylphosphonoacétate d'éthyle en solution dans 200 cm³ de diméthoxy-1,2.éthane à une suspension de 14,4 g d'hydrure de sodium dans 300 cm³ de diméthoxy-1,2 éthane à une température voisine de 10 °C. On ajoute ensuite goutte à goutte et à une température voisine de 10°C une solution de 99 g de nitro-4 acétophénone dans 500 cm³ de diméthoxy-1,2 éthane, puis agite 3 h à une température voisine de 20 °C. Le mélange réactionnel est ensuite versé dans 3000 cm³ d'eau distillée et extrait 3 fois par 1500 cm³ au total de chlorure de méthylène; les phases organiques sont réunies et lavées par 500 cm³ d'eau distillée, séchées sur sulfate de magnésium et concentrées à sec sous pression réduite (2,7 kPa) à 40 °C. Le résidu obtenu est dissous dans 100 cm³ de chlorure de méthylène et la solution obtenue est versée sur 1500 g de gel de silice contenus dans une colonne de 7,5 cm de diamètre. On élue d'abord par 3400 cm³ de chlorure de méthylène et l'éluat correspondant est éliminé; on élue ensuite par 5000 cm³ de chlorure de méthylène et l'éluat correspondant est concentré à sec sous pression réduite (2,7 kPa) à 40 °C. On obtient ainsi 95,8 g de (nitro-4 phényl)-3 butène-2-oate d'éthyle sous forme d'une huile brune [spectre IR (max en cm-¹): 1720 et 1130 (COOC₂H₅), 1525 et 1345 (-N0₂ aromatique), 1635 (double liaison conjuguée)].

### Exemple 21

En opérant d'une manière analogue à celle décrite à l'exemple 1, mais à partir de 6,7 g de dichloro-4,5 dithiole-1,2 one-3 et de 6,5 g d'(amino-4 phényl)-3 méthoxy-1 butane, on obtient, après recristallisation dans le cyclohexane, 7,5 g de chloro-4 [(méthoxy-1 butyl-3)-4 phénylamino]-5 dithiole-1,2 one-3 fondant à 95 °C.

L'(amino-4 phényl)-3 méthoxy-1 butane peut être préparé de la manière suivante: on agite une suspension de 17,3 g de méthoxy-1 (tritylamino-4 phényl)-3-butane dans 350 cm³ de méthanol contenant 5,3 cm³ d'acide chlorhydrique concentré (d = 1,19) à une température voisine de 20 °C pendant 20 h. Le mélange réactionnel est ensuite versé dans 1500 cm³ d'eau distillée et le mélange obtenu est acidifié à pH 2 par addition d'acide chlorhydrique aqueux 4N, puis est extrait 3 fois par 750 cm³ au total d'éther éthylique. La phase aqueuse est alcalinisée jusqu'à pH 9 par addition de soude aqueuse 4N, puis extraite 2 fois par 400cm³ au total d'éther éthylique. Les phases organiques sont réunies, séchées sur sulfate de magnésium, filtrées et concentrées à sec sous pression réduite (2,7 kPa) à 40 °C. On obtient ainsi 6,6 g d'(amino-4 phényl)-3 méthoxy-1 butane sous forme d'huile orangée [Rf = 0,5; chromatographie sur couche mince de gel de silice; éluant: chlorure de méthylène/acétate. d'éthyle (90/10 en volumes)].

Le méthoxy-1 (tritylamino-4 phényl)-3 butane peut être préparé en ajoutant 1,8 g d'hydrure de sodium à une solution de 27,7 g de (tritylamino-4 phényl)-3 butanol et de 11,4 g d'iodure de méthyle dans 40 cm³ de diméthoxy-1,2 éthane à une température voisine de 20 °C. Le mélange réactionnel est agité pendant 20 h à une température voisine de 20 °C puis est concentré à sec sous une pression voisine de 100 kPa à une température de 100°C. Le solide obtenu est lavé 4 fois par 400 cm³ au total d'eau distillée, séché à l'air à une température voisine de 20 °C puis recristallisé dans un mélange d'oxyde d'isopropyle et d'acétate d'éthyle (75/25 en volume). On obtient ainsi 17,3 g de méthoxy-1 (tritylamino-4 phényl)-3 butane fondant à 141 °C.

Le (tritylamino-4 phényl)-3 butanol peut être préparé de la manière suivante: on ajoute goutte à goutte une solution de 35,2 g de (tritylamino-4 phényl)-3 butyrate d'éthyle dans 60 cm³ de tétrahydrofuranne à une suspension de 5,1 g de boro- hydrure de potassium et de 4 g de chlorure de lithium dans 80 cm³ de tétrahydrofuranne à une température voisine de 20 °C. Le mélange réactionnel est ensuite agité au reflux pendant 6 h, puis refroidi à une température voisine de 10 °C; on ajoute alors 70 cm³ d'eau distillée, puis acidifie à un pH voisin de 3 par addition de 10 cm³ d'acide chlorhydrique concentré (d = 1,19) et enfin alcalinise par addition de 15 cm³ de soude aqueuse 3,3 N. La phase organique est décantée, séchée sur sulfate de magnésium puis concentré à sec sous pression réduite (2,7 kPa) à 40 °C. Après lavage du résidu par 120 cm³ d'éther de pétrole et séchage sous pression réduite (2,7 kPa), on obtient 27,7 g de (tritylamino-4 phényl)-3 butanol fondant à 114°C.

Le (tritylamino-4 phényl)-3 butyrate d'éthyle peut être préparé de la manière suivante: on ajoute goutte à goutte une solution de 10,1 g de triéthylamine dans 20 cm³ de diméthylformamide à une solution de 20,7 g d'(amino-4 phényl)-3 butyrate d'éthyle et de 28,8 g de chlorure de tri- phénylméthyle dans 80 cm³ de diméthylformamide à une température voisine de 30 °C. Le mélange réactionnel est agité pendant 3 h à une température voisine de 20 °C, puis versé dans 1000 cm³ d'eau distillée; l'émulsion obtenue est extraite par 350 cm³ de chloroforme, la phase organique est lavée par 50 cm³ d'eau distillée, séchée sur sulfate de magnésium, filtrée et concentrée sous pression réduite (0,7 kPa) à 60 °C. Après lavage de résidu par 60 cm³ de méthanol et séchage, on obtient 35,2 g de (tritylamino-4 phényl)-3 butyrate d'éthyle fondant à 130 °C.

### Exemple 22

En opérant de manière analogue à celle décrite à l'exemple 17, mais à partir de 12,9 g de dichloro-4,5 dithiole-1,2 one-3 et de 15,3 g d'amino-4 benzylcarbamate de tert-butyle, on obtient 25,3 g de [(chloro-4 oxo-3 dithiole-1,2 yl-5 amino]-4 benzylcarbamate de tert-butyle fondant à 80 °C.

Pour libérer la fonction amine de son groupement protecteur, on opère de la manière suivante: on verse goutte à goutte en 30 min 250 cm³ d'une solution 2N d'acide chlorhydrique gazeux dans l'acide acétique sur le [(chloro-4 oxo-3 dithiole-1,2 yl-5) amino-4] benzylcarbamate de tert-butyle obtenu précédemment. La suspension obtenue est agitée pendant 6 h à une température voisine de 20 °C puis le produit insoluble est séparé par filtration, lavé 3 fois par 120 cm³ au total d'éther isopropylique puis séché sous pression réduite (2,7 kPa). Après recristallisation dans l'eau, on obtient 5,4 g de chlorhydrate d'(aminométhyl-4 phénylamino)-5 chloro-4 dithiole-1,2 one-3 fondant à 291 °C.

L'amino-4 benzylcarbamate de tert-butyle peut être préparé par hydrogénation catalytique de 18,1 g de (nitro-4 benzyl)carbamate de tert-butyle dans 650 cm³ d'éthanol en présence de 0,75 g de palladium à 10% sur noir de carbone, sous une pression voisine de 105 kPa, à une température voisine de 25 °C pendant 45 min. Après séparation du catalyseur par filtration, le mélange réactionnel est concentré à sec sous pression réduite (2,7 kPa) à 40 °C. On obtient ainsi 15,4 g d'amino-4 benzylcarbamate de tert]-butyle fondant à 77 °C.

Le (nitro-4 benzyl) carbamate de tert-butyle peut être préparé en additionnant en 30 min 14,2 g de ditertiobutyldicarbonate en solution dans 35 cm³ d'acétate d'éthyle à une suspension de 12,3 g de chlorhydrate de nitro-4 benzylamine dans 65cm³ d'acétate d'éthyle contenant 6,6 g de triéthylamine. Le mélange réactionnel est agité 16 h à une température voisine de 20 °C, puis est versé dans 100 cm³ d'eau distillée; la phase organique est décantée, lavée 3 fois par 150 cm³ au total d'eau distillée, séchée sur sulfate de magnésium, filtrée puis concentrée à sec sous pression réduite (2,7 kPa) à 40 °C. On obtient ainsi 15,7 g de (nitro-4 benzyl) carbamate de tert-butyle fondant à 110°C.

### Exemple 23

En opérant d'une manière analogue à celle décrite à l'exemple 1 mais à partir de 104,5 g de dichloro-4,5 dithiole-1,2 one-3 et de 132,2 g d'(amino-4-phényl)-2 éthylcarbamate de tert-butyle, on obtient 150,6 g de [(chloro-4 oxo-3-dithiole-1,2 yl-5) amino-4 phényl]-2 éthylcarbamate de tert-butyle fondant à 147 °C.

Pour libérer la fonction amine de son groupement protecteur, on opère d'une manière analogue à celle décrite à l'exemple 22, mais à partir de 38,6 g de [(chloro-4 oxo-3 dithiole-1,2 yl-5) amino-4 phényl]-2-éthylcarbamate de tert-butyle. On obtient ainsi, après recristallisation dans un mélange de méthanol et d'éthanol (50/50 en volumes), 23 g de chlorhydrate d'[(amino-2 éthyl)-4 phénylamino]-5 chloro-4 dithiole-1,2 one-3 fondant à 164 °C.

L'(amino-4 phényl)-2 éthylcarbamate de tert-butyle peut être obtenu en additionnant en 1 h une solution de 122 g d'anhydride de l'acide tert-butoxycarboxylique dans 920 cm³ d'acétate d'éthyle à une solution de 76,1 g d'(amino-2 éthyl)-4 aniline dans 200 cm³ d'acétate d'éthyle. Le mélange réactionnel est agité ensuite pendant 3 h à une température voisine de 20 °C, puis concentré à sec sous pression réduite (2,7 kPa) à 40 °C. On obtient ainsi 118 g d'(amino-4 phényl)-2 éthylcarbamate de tert-butyle fondant à 71 °C.

L'(amino-2 éthyl)-4 aniline peut être obtenue de la manière suivante: on ajoute goutte à goutte une solution de 200 g de chlorure d'aluminium dans 2000 cm³ de tétrahydrofuranne à une suspension de 57 g d'hydrure de lithium et d'aluminium dans 1500 cm³ de tétrahydrofuranne à une température voisine de 15 °C. On ajoute ensuite goutte à goutte et à une température voisine de 20 °C, une solution de 99 g d'(amino-4 phényl) acétonitrile dans 500 cm³ de tétrahydrofuranne. Le mélange réactionnel est agité à 20 °C environ pendant 20 h, puis refroidi à une température voisine de 5 °C. On ajoute alors goutte à goutte et successivement 128 cm³ d'eau distillée, 98cm³ d'une solution aqueuse de soude 6N, 450cm³ d'eau distillée et enfin 340cm³ d'une solution aqueuse de soude 10 N. Le précipité formé est séparé par filtration; le filtrat est séché sur du sulfate de sodium puis concentré à sec sous pression réduite (2,7 kPa) à 50 °C. Par distillation du résidu sous pression réduite, on obtient 83,3g d'(amino-2 éthyl)-4 aniline sous forme d'huile incolore bouillant à 138-142 °C sous 0,47 kPa.

L'(amino-4 phényl) acétonitrile peut être préparé par hydrogénation catalytique de 121,6 g de (nitro-4 phényl) acétonitrile dans 2000 cm³ d'éthanol en présence de 7,5 g de palladium à 5% sur noir de carbone sous une pression voisine de 100 kPa à une température voisine de 30 °C pendant 1 h. Après séparation du catalyseur par filtration, le mélange réactionnel est concentré à sec sous pression réduite (2,7 kPa) à 40 °C, on obtient 98,9 g d'(amino-4 phényl) acétonitrile fondant à 42 °C.

### Exemple 24

A une solution de 59,4 g de [(chloro-4 oxo-3 dithiole-1,2 yl-5) amino-4-phényl]-2 éthylcarbamate de tert-butyle, préparé comme à l'exemple 23, dans 475 cm³ de tétrahydrofuranne à une température voisine de 15 °C, on ajoute goutte à goutte 49 g d'hydrure de tributylétain. Le mélange réactionnel est ensuite agité pendant 20 h à une température voisine de 20 °C puis est versé dans 1500 cm³ d'eau distillée. On ajoute alors 200 cm³ de chlorure de méthylène et décante la phase aqueuse. Celle-ci est extraite de nouveau par 200cm³ de chlorure de méthylène; les phases organiques sont réunies, lavées par 200 cm³ d'eau distillée, séchées sur sulfate de magnésium filtrées et concentrées à sec sous pression réduite (2,7 kPa) à 50 °C. Le résidu est dissous dans 100cm³ de chlorure de méthylène et la solution obtenue est versée sur 1500 g de gel de silice contenus dans une colonne de 7,5 cm de diamètre. On élue d'abord par 10 litres d'un mélange de chlorure de méthylène et d'acétate d'éthyle (93/7 en volumes) puis par 10 d'un mélange de chlorure de méthylène et d'acétate d'éthyle (88/12 en volumes);les éluats correspondant sont éliminés. On élue ensuite par 11 d'un mélange de chlorure' de méthylène et d'acétate d'éthyle (85/15 en volumes) et l'éluat correspondant est concentré à sec sous pression réduite (2,7 kPa) à 40 °C. On obtient 29,3g d'[oxo-3 dithiole-1,2 yl-5) amino-4 phényl]-2 éthylcarbamate de tert-butyle sous forme d'huile brune [Rf = 0,28; chromatographie sur couche mince de gel de silice; éluant: chlorure de méthylène/acétate d'éthyle (80/20 en volumes)].

Pour libérer l'amine de son groupement protecteur on opère d'une manière analogue à celle décrite à l'exemple 22, mais à partir de 12 g d'[oxo-3 dithiole-1,2 yl-5) amino-4 phényl]-2 éthyl carbamate de tert-butyle. On obtient ainsi, après recristallisation dans l'éthanol, 2,5 g de chlorhydrate d'[(amino-2 éthyl)-4 phénylamino]-5 dithiole-1,2 one-3 fondant à 228 °C.

### Exemple 25

En opérant d'une manière analogue à celle décrite à l'exemple 17 mais à partir de 96,5 g de dichloro-4,5 dithiole-1,2 one-3 et de 137 g d'[(amino-4 phényl)-2 propyl] carbamate de tert-butyle, on obtient 206 g de [(chloro-4 oxo-3 dithiole-1,2 yl-5) amino-4 phényl]-2 propylcarbamate de tert-butyle. [Rf = 0,32, chromatographie sur couche mince de gel de silice; éluant: chlorure de méthylène/acétate d'éthyle (90/10 en volumes)].

Pour libérer l'amine de son groupement protecteur, on opère de manière analogue à celle décrite à l'exemple 22, mais à partir de 110 g de [(chloro-4 oxo-3 dithiole-1,2 yl-5) amino-4 phényl]-2 propylcarbamate de tert-butyle. On obtient ainsi, après recristallisation dans le méthanol, 41 g de chlorhydrate d'[(amino-1 propyl-2)-4 phénylamino]-5 chloro-4 dithiole-1,2 one-3 fondant à 194 °C.

L'[(amino-4 phényl)-2 propyl] carbamate de tert-butyle peut être préparé d'une manière analogue à celle décrite à l'exemple 23. A partir de 88,5g g d'(amino-4 phényl)-2 propylamine et de 111 g d'anhydride de l'acide tert-butoxycarboxylique, on obtient 135,7 g d'(amino-4 phényl-2) propylcarbamate de tert-butyle sous forme d'huile brune. [Rf = 0,20; chromatographie sur couche mince de gel de silice; éluant: chlorure de méthylène/acétate d'éthyle (90/10 en volumes)].

L'(amino-4 phényl)-2 propylamine peut être préparée par une méthode analogue à celle décrite à l'exemple 23. A partir de 80,3 g d'(amino-4 phényl)-2 propionitrile, on obtient 75,2 g d'(amino-4 phényl)-2 propylamine sous forme d'huile brune. [Rf = 0,50; chromatographie sur couche mince de gel de silice; éluant: méthanol/ammoniaque concentré (d = 0,92) (99/1 en volumes)].

L'(amino-4 phényl)-2 propionitrile peut être préparé par une méthode analogue à celle décrite à l'exemple 23. A partir de 185 g de (nitro-4 phényl)-2 propionitrile, on obtient 155 g d'(amino-4 phényl)-2 propionitrile sous forme d'huile brune. [Rf = 0,26; chromatographie sur couche mince de gel de silice; éluant: chlorure de méthylène]. Le (nitro⁼4 phényl)-2 propionitrile peut être préparé selon la méthode décrite par S. Opolski, Z. Ko- watski et J. Pilewski Chem. Ber. 49,2276 (1916).

### Exemple 26

Une suspension de 11,8 g d'(amino-4 phénylamino)-5 chloro-4 dithiole-1,2 one-3 dans 30 cm³ de diméthyl acétal de diméthylformamide est agitée pendant 3 h à une température voisine de 25 °C. Le produit insoluble est séparé par filtration, lavé 3 fois par 75 cm³ au total d'éther isopropylique puis séché sous pression réduite (2,7 kPa). Le produit obtenu est dissous dans 300 cm³ d'éthanol; on ajoute à cette solution 10 cm³ d'une solution 3 N d'acide chlorhydrique gazeux dans l'éther éthylique. Le produit insoluble est séparé par filtration, lavé 2 fois par 30 cm³ au total d'éthanol puis séché sous pression réduite (2,7 kPa). Après recristallisation dans un mélange d'eau et de diméthylformamide (50/50 en volumes), on obtient 2,2 g de chlorhydrate de chloro-4 (diméthylamino- méthylèneamino-4 phényl)-5 dithiole-1,2 one-3 fondant à 270 °C.

L'(amino-4 phénylamino)-5 chloro-4 dithiole-1,2 one-3 peut être préparé par la méthode décrite dans le brevet français 1498374.

### Exemple 27

En opérant d'une manière analogue à celle décrite à l'exemple 26, mais à partir de 4,1 g d'(ami- nométhyl-4 phénylamino)-5 chloro-4 dithiole-1,2 one-3, on obtient, après recristallisation dans un mélange de méthanol et d'éther isopropylique (70/30 en volumes), 1,45 g de chlorhydrate de chloro-4 (diméthylaminométhylène aminométhyl-4 phénylamino)-5 dithiole-1,2 one-3 fondant à 155°C.

L'(aminométhyl-4 phénylamino)-5 chloro-4 dithiole-1,2 one-3 peut être préparée comme à l'exemple 22.

### Exemple 28

En opérant d'une manière analogue à celle décrite à l'exemple 2 mais à partir de 63,5 g de dichloro-4,5 dithiole-1,2 one-3 et de 89,8 g d'(amino-4 phényl)-2 butylcarbamate de tert-butyle, on obtient 80,5 g de [(chloro-4 oxo-3 dithiole-1,2 yl-5) amino-4 phényl]-2 butylcarbamate de tert-butyle sous forme d'huile rouge. [Rf = 0,47; chromatographie sur couche mince de gel de silice, éluant: chlorure de méthylène (acétate d'éthyle (90/10 en volumes)].

Pour libérer l'amine de son groupement protecteur, on opère de manière analogue à celle décrite à l'exemple 22, mais à partir de 80 g de [(chloro-4 oxo-3 dithiole-1,2 yl-5) amino-4 phényl]-2 butylcarbamate de tert-butyle. On obtient ainsi, après recristallisation dans l'éthanol, 24,5 g de chlorhydrate d'(amirio-1 butyl-2)-4 phénylamino]-5 chloro-4 dithiole-1,2 one-3 fondant à 230 °C.

L'(amino-4 phényl)-2 butylcarbamate de tert-butyle peut être préparé d'une manière analogue à celle décrite à l'exemple 23. A partir de 56,5 g d'(amino-4 phényl)-2 butylamine etde74,7 g d'anhydride de l'acide tert-butoxycarboxylique, on obtient 89,8 g d'(amino-4 phényl)-2 butylcarbamate de tert-butyle sous forme d'huile rouge [Rf = 0,22; chromatographie sur couche mince de gel de silice; éluant: chlorure de méthylène/acétate d'éthyle (90/10 en volumes)].

L'(amino-4 phényl)-2 butylamine peut être préparée d'une manière analogue à celle décrite à l'exemple 23. A partir de 64 g d'(amino-4 phényl)-2 butyronitrile, on obtient 56,5 g d'(amino-4 phényl)-2 butylamine sous forme d'huile brune [Rf = 0,25; chromatographie sur couche mince de gel de silice; éluant: méthanol/ammoniaque concentré (d = 0,92) (99/1 en volumes)].

L'(amino-4 phényl)-2 butyronitrile peut être préparé par une méthode analogue à celle décrite à l'exemple 23. A partir de 149 g de (nitro-4 phényl)-2 butyronitrile, on obtient 127 g d'(amino-4 phényl)-2 butyronitrile sous forme d'huile jaune. [Rf = 0,21; chromatographie sur couche mince de gel de silice; éluant: chlorure de méthylène].

Le (nitro-4 phényl)-2 butyronitrile peut être préparé selon la méthode décrite par E. Fourneau et G. Sandulesco, Bull. Soc. Chim. France 41, 450, (1927).

### Exemple 29

En opérant d'une manière analogue à celle décrite à l'exemple 24 mais à partir de 61,7 g de [(chloro-4 oxo-3 dithiole-1,2 yl-5) amino-4 phényl]-2 propyl-1 carbamate de tert-butyle et de 49 g d'hydrure de tributylétain, on obtient 14,2 g d'[(oxo-3 dithiole-1,2 yl-5) amino-4 phényl]-2 propylcarbamate de tert-butyle sous forme d'huile brune [Rf = 0,41; chromatographie sur couche mince de gel de silice; éluant: chlorure de méthylène/acétate d'éthyle (80/20 en volumes)].

Pour libérer l'amine de son groupement protecteur, on opère de manière analogue à celle décrite à l'exemple 22, mais à partir de 14,2 g d'[(oxo-3 dithiole-1,2 yl-5) amino-4 phényl]-2 propylcarbamate de tert-butyle. On obtient ainsi 6,7 g de chlorhydrate d'[(amino-1 propyl-2)-4 phénylamino]-5 dithiole-1,2 one-3 fondant à 148 °C.

### Exemple 30

En opérant d'une manière analogue à celle décrite à l'exemple 1, mais à partir de 12,8 g de dichloro-4,5 dithiole-1,2 one-3 et de 16,8 g d'[(amino-4 phényl)-1 propyl-2] carbamate de tert-butyle, on obtient 14,7 g de [(chloro-4 oxo-3 dithiole-1,2 yl-5) amino-4 phényl]-1 propyl-2 carbamate de tert-butyle fondant à 155 °C.

Pour libérer l'amine de son groupement protecteur, on opère d'une manière analogue à celle décrite à l'exemple 22, mais à partir de 14,2 g de [(chloro-4 oxo-3 dithiole-1,2 yl-5) amino-4 phényl]-1 propyl-2 carbamate de tert-butyle. On obtient ainsi, après trituration dans l'oxyde d'isopropyle, 9,5 g de chlorhydrate d'[(amino-2 propyl)-4 phénylamino]₋5 chloro-4 dithiole-1,2 one-3 fondant à 149°C.

L'[(amino-4-phényl)-1 propyl-2] carbamate de tert-butyle peut être préparé d'une manière analogue à celle décrite à l'exemple 23. A partir de 18,4 g d'(amino-4 phényl)-1 propylamine-2 et de 26,7 g d'anhydride de l'acide tert-butoxycarboxylique, on obtient 20 g d'[(amino-4 phényl)-1 propyl-2] carbamate de tert-butyle sous forme d'huile rouge [Rf = 0,20; chromatographie sur couche mince de gel de silice; éluant: chlorure de méthylène/acétate d'éthyle (90/10 en volumes)].

L'(amino-4 phényl-1)-1 propylamine-2 peut être préparée de la manière suivante: on ajoute goutte à goutte une solution de 26,3 g d'un mélange d'(amino-4 phényl)-1 nitro-2 propène-1 et d'(amino-4 phényl)-1 nitro-2 propane dans 200 cm³ de diméthoxy-1,2 éthane à 440 cm³ d'une solution toluénique à 70% d'hydrure de bis (méthoxy-2 éthoxy) aluminium et de sodium. Le mélange réactionnel est ensuite chauffé au reflux pendant 1 h puis refroidi à une température voisine de 10 °C. On ajoute ensuite goutte à goutte 250 cm³ d'eau distillée, puis 250 cm³ d'éther éthylique. Le produit insoluble est séparé par filtration, le filtrat est décanté et la phase aqueuse est extraite 3 fois par 900 cm³ au total d'éther éthylique, puis 3 fois par 900 cm³ au total de chloroforme. Les phases organiques sont réunies, séchées sur carbonate de potassium, filtrées puis concentrées à sec sous pression réduite (2,7 kPa) à 50 °C. On obtient ainsi 18,4 g d'(amino-4 phényl)-1 propylamine-2 sous forme d'huile brune [Rf = 0,12; chromatographie sur couche mince de gel de silice; éluant: méthanol/ammoniaque concentrée (d = 0,92) (99/1 en volumes)].

Le mélange d'(amino-4 phényl)-1 nitro-2 propène-1 et d'(amino-4 phényl)-1 nitro-2 propane peut être obtenu par hydrogénation catalytique de 47 g de nitro-2 (nitro-4 phényl)-1 propène-1 dans 1500 cm³ d'éthanol en présence de 5 g de palladium à 5% sur noir de carbone sous une pression voisine de 100 kPa à une température voisine de 50 °C pendant 1,5 h. Après séparation du catalyseur par filtration, le mélange réactionnel est concentré à sec sous pression réduite (2,7 kPa) à 50 °C. On obtient 30 g de mélange d'(amino-4 phényl)-1 nitro-2 propène-2 et d'(amino-4 phényl)-1 nitro-2 propane sous forme d'une huile brune [Rf = 0,81 et 0,54; chromatographie sur couche mince de gel de silice; éluant: chlorure de méthylène/acétate d'éthyle (50/50 en volumes)].

Le nitro-2 (nitro-4 phényl)-1 propène-1 peut être préparé selon la méthode décrite par B. Priebs Liebig's Ann. 225, 319 (1884).

### Exemple 31

En opérant d'une manière analogue à celle décrite à l'exemple 2 mais à partir de 5 g de dichloro-4,5 dithiole-1,2 one-3 et de 8,6 g de (méthylamino-4 phényl)-2 propylcarbamate de tert-butyle, on obtient 6,7 g de [N-(chloro-4 oxo-3 dithiole-1,2 yl-5) N-méthylamino-4 phényl]-2 propylcarbamate de tert-butyle sous forme d'une huile jaune [Rf = 0,59; chromatographie sur couche mince de gel de silice; -éluant: chlorure de méthylène/acétate d'éthyle (90/10 en volumes)].

Pour libérer l'amine de son groupement protecteur, on opère d'une manière analogue à celle décrite à l'exemple 22, mais à partir de 6,8 g de [N-(chloro-4 oxo-3 dithiole-1,2 yl-5) N-méthylamino-4 phényl]-2 propylcarbamate de tert-butyle. On obtient ainsi, après trituration dans l'oxyde d'isopro- pyle, 4 g de chlorhydrate de N-[(amino-1 propyl-2)-4 phényl] N-méthylamino-5 chloro-4 dithiole-1,2⁼one-3 fondant à 190 °C.

Le (méthylamino-4 phényl)-2 propylcarbamate de tert-butyle peut être préparé selon une méthode analogue à celle décrite à l'exemple 23. A partir de 9,9 g de (méthylamino-4 phényl)-2 propylamine et de 11,5 g d'anhydride de l'acide tert-butoxycarboxylique, on obtient 8,6 g de [(méthylamino-4 phényl)-2 propyl] carbamate de tert-butyle sous forme d'huile jaune [Rf = 0,5; chromatographie sur couche mince de gel de silice; éluant: chlorure de méthylène/acétate d'éthyle (90/10 en volumes)].

La (méthylamino-4 phényl)-2 propylamine peut être préparée de la manière suivante: on ajoute goute à goutte une solution de 13,9 g de (formylamino-4 phényl)-2 propionitrile dans 20 cm³ de tétrahydrofuranne à une suspension de 12,2 g d'hydrure de lithium et d'aluminium dans 120 cm³ de tétrahydrofuranne, à une température voisine de 65 °C. Le mélange réactionnel est ensuite agité pendant 2 h au reflux puis pendant 16 h à une température voisine de 20 °C. On ajoute ensuite goutte à goutte 12 cm³ d'eau distillée, 24 cm³ d'une solution aqueuse de soude 4 N puis 48 cm³ d'eau distillée. Le produit insoluble est séparé par filtration, et le filtrat est versé dans un mélange de 100 cm³ d'une solution aqueuse d'acide chlorhydrique 4N et de 100 cm³ de chlorure de méthylène. La phase organique est décantée; la phase aqueuse est lavée par 100 cm³ de chlorure de méthylène, puis alcalinisée par addition de 50 cm³ d'une solution aqueuse de soude 10 N. L'émulsion obtenue est extraite 3 fois par 300 cm³ au total de chlorure de méthylène; les phases organiques sont réunies, séchés sur du carbonate de potassium, filtrées et concentrées à sec sous pression réduite (2,7 kPa) à 50 °C. On obtient ainsi 10,4 g de (méthylamino-4 phényl)-2 propylamine sous forme d'huile brune [Rf = 0,50; chromatographie sur couche mince de gel de silice; éluant: méthanol/ammoniaque (99/1 en volumes)].

Le (formylamino-4 phényl)-2 propionitrile peut être préparé de la manière suivante: on chauffe pendant 2 h à une température voisine de 55 °C un mélange de 5,7 cm³ d'acide formique et de 14,2 cm³ d'anhydride acétique puis, après refroidissement à une température voisine de 25 °C, on ajoute goutte à goutte 14,6 g d'(amino-4 phényl)-2 propionitrile. Le mélange réactionnel est ensuite agité pendant 20 h à une température voisine de 20 °C, puis on ajoute 30 cm³ de chlorure de méthylène et la solution obtenue est lavée 2 fois par 60 cm³ au total d'eau distillée, 2 fois par 60 cm³ au total d'une solution aqueuse saturée de bicarbonate de sodium puis séchée sur sulfate de magnésium, filtrée et concentrée sous pression réduite (2,7 kPa) à 50 °C. On obtient ainsi 14,4 g de (formylamino-4 phényl)-2 propionitrile sous forme d'huile rouge [Rf = 0,50; chromatographie sur couche mince de gel de silice; éluant: chlorure de méthylène/acétate d'éthyle (50/50 en volumes)].

### Exemple 32.

En opérant d'une manière analogue à celle décrite à l'exemple 1, mais à partir de 39,5 g de dichloro-4,5 dithioie-1,2 one-3 et de 34,6 g d'(amino-4 phényl)-2 propionamide, on obtient, après recristallisation dans un mélange d'acétonitrile et d'acétate d'éthyle (75/25 en volumes), 32,8 g de [(chloro-4 oxo-3 dithiole-1,2 yl-5) amino-4 phényl]-2 propionamide fondant à 195 °C.

L'(amino-4 phényl)-2 propionamide peut être préparée par hydrogénation catalytique de 51,4 g de (nitro-4 phényl)-2 propionamide dans 700 cm³ d'éthanol en présence de 2,2 g de palladium à 5% sur noir de carbone, à une température voisine de 35 °C, sous une pression voisine de 100 kPa pendant 1 h. Après séparation du catalyseur par filtration, le mélange réactionnel est concentré à sec sous pression réduite (2,7 kPa) à 40 °C. Le résidu obtenu est lavé 2 fois par 200 cm³ au total d'oxyde d'isopropyle puis séché à l'air à une température voisine de 20 °C. On obtient ainsi 34,6 g d'(amino-4 phényl)-2 propionamide fondant à 126 °C.

La (nitro-4 phényl)-2 propionamide peut être préparée de la manière suivante: on chauffe une suspension de 52,8 g de (nitro-4 phényl)-2 propionitrile dans 150 cm³ d'acide chlorhydrique concentré (d = 1,19) pendant 1,5 h à une température voisine de 60 °C. On refroidit la solution obtenue à une température voisine de 10 °C, puis on y ajoute 150 cm³ d'eau distillée. Le précipité apparu est séparé par filtration, lavé 3 fois par 300 cm³ au total d'eau distillée, 2 fois par 300 cm³ au total d'une solution aqueuse saturée de bicarbonate de sodium, 2 fois par 200 cm³ au total d'eau distillée, puis séché à l'air à une température voisine de 20 °C. On obtient ainsi 48,8 g de (nitro-4 phényl)-2 propionamide fondant à 117 °C.

### Exemple 33

A une solution de 16,8 g de chlorhydrate d'[(amino-1 propyl-2)-4 phénylamino]-5 chloro-4 dithiole-1,2 one-3 dans 100 cm³ de pyridine, on ajoute, à une température de -5 °C, 3,6 cm³ de chlorure d'acétyle. Le mélange réactionnel est maintenu pendant 10 min à une température voisine de 0 °C. On ajoute ensuite à cette même température une solution de 7 cm³ de triéthylamine dans 50 cm³ de pyridine. Le mélange réactionnel est maintenu pendant 45 min à une température voisine de 20 °C. On ajoute alors 450 cm³ d'éther diéthylique et, sépare par décantation la liqueur surnageante de la fraction insoluble. La phase organique ainsi obtenue est lavée par 100 cm³ d'eau distillée, traitée par 1 g de noir décolorant, séchée sur sulfate de sodium, filtrée, et évaporée à sec sous pression réduite (2,7 kPa). Par recristallisation du résidu ainsi obtenu dans 10 cm³ d'acétonitrile, on obtient 4,9 g d'[(acétyla- mino-1 propyl-2)-4 phénylamino]-5 chloro-4 dithiole-1,2 one-3 fondant à 179 °C [Rf = 0,4 chromatographie sur couche mince de silice; éluant: mélange chloroforme/méthanol 80/20 (en volumes)].

### Exemple 34

En opérant de manière analogue à celle décrite à l'exemple 33, mais à partir de 10,1 g de chlorhydrate d'[(amino-1 propyl-2)-4 phénylamino]-5 chloro-4 dithiole-1,2 one-3, et de 2,9 cm³ de chlo- roformiate d'éthyle, on obtient 7,5 g d'une huile brune. Cette huile est dissoute dans 40 cm³ de chlorure de méthylène, et la solution obtenue est versée sur 150 g de gel de silice contenus dans une colonne de 2,5 cm de diamètre. On élue d'abord par 500 cm³ de chlorure de méthylène, et l'éluat correspondant est éliminé. On élue ensuite par 1400 cm³ de chlorure de méthylène, et l'éluat correspondant est évaporé à sec sous pression réduite (2,7 kPa), à une température voisine de 40 °C. Le résidu ainsi obtenu est dissous dans 50 cm³ de chlorure de méthylène et la solution obtenue est lavée par un mélange de 20 cm³ d'eau et 4 cm³ d'une solution aqueuse d'acide chlorhydrique 0,1 N, puis par 20 cm³ d'eau. La phase organique est séparée par décantation, séchée sur sulfate de sodium, filtrée, et évaporée à sec sous pression réduite (2,7 kPa), à une température voisine de 60 °C.

On obtient ainsi 3,7 g de [(chloro-4 oxo-3 dithiole-1,2 yl-5) amino-4 phényl)]-2 propylcarbamate d'éthyle sous forme d'une meringue jaune [Rf = 0,5 chromatographie sur couche mince de silice; éluant: chloroforme/méthanol: 80/20 (en volumes)].

### Exemple 35

Une suspension de 12,0 g d'[(amino-1 propyl-2)-4 phénylamino]-5 chloro-4 dithiole-1,2 one-3 dans 300 cm³ de formiate de méthyle est agitée à une température voisine de 25 °C pendant 75 h. On filtre ensuite le mélange réactionnel et lave l'insoluble par 150 cm³ de formiate de méthyle. Les filtrats sont réunies, et la solution organique ainsi obtenue est évaporée à sec sous pression réduite (2,7 kPa) à une température voisine de 40.°C. Par recristallisation du résidu ainsi obtenu dans 60 cm³ d'acétonitrile, on obtient 5,0 g de chloro-4 [(Formylamino-1 propyl-2)-4 phénylamino]-5 dithiole-1,2-one-3 fondant à 143 °C.

[Rf = 0,4 chromatographie sur couche mince de silice; éluant: chloroforme/méthanol 80/20 (en volumes)].

L'[(amino-1 propyl-2)-4 phénylaminoj-5 chloro-4 dithiole-1,2 one-3 est préparée de la manière suivante: à une solution de 10,1 g de chlorhydrate d'[(amino-1 propyl-2)-4 phénylaminol-5 chloro-4 dithiole-1,2 one-3 dans 90 cm³ d'eau on ajoute à une température voisine de 20 °C 25 cm³ d'une solution de soude N. Les cristaux apparus sont séparés par filtration, lavés trois fois par 90 cm³ au total d'eau, et séchés à l'air ambiant pendant 24 h. On obtient ainsi 7,2 g d'[(amino-1 propyl-2)-4 phénylamino]-5 chloro-4 dithiole-1,2 one-3 fondant à 120 °C. [Rf = 0,3 chromatographie sur couche mince de silice; éluant: chloroforme/méthanol 80/ 20 (en volumes)].

### Exemple 36

A une suspension de 6,0 g d'[(amino-1 propyl-2)-4 phénylamino]-5 chloro-4 dithiole-1,2 one-3 dans 100 cm³ de chlorure de méthylène, on ajoute, à une température voisine de 20 °C une solution de 1,1 g d'isocyanate de méthyle dans 25 cm³ de chlorure de méthylène. Le mélange réactionnel est maintenu pendant 30 min à une température voisine de 20 °C. Le précipité formé est séparé par filtration, et lavé 3 fois par 50 cm³ au total de méthanol, puis séché à l'air ambiant pendant 16 h. Par recristallisation du solide ainsi obtenu dans 60 cm³ d'éthanol, on obtient 3,4 g de N-[(chloro-4 oxo-3 dithiole-1,2 yl-5) amino-4 phényl]-2N'-méthyl-propylurée fondant à 176 °C.

[Rf = 0,4 chromatographie sur couche mince de silice; éluant: chloroforme/méthanol 80/20 (en volumes)].

### Exemple 37

En opérant d'une manière analogue à celle décrite à l'exemple 24, mais à partir de 28,6 g de chloro-4 (isopropyl-4 phénylamino)-5 dithiole-1,2 one-3 et de 32 g d'hydrure de tributylétain, on obtient, après recristallisation dans un mélange d'éther isopropylique et d'acétate d'éthyle (70/30 en volumes), 6,3 g d'(isopropyl-4-phénylamino)-5 dithiole-1,2 one-3 fondant à 114 °C.

La chloro-4 (isopropyl-4 phénylamino)-5 dithiole-1,2 one-3 peut être préparée selon le méthode décrite dans le brevet français 1498374.

### Exemple 38

A une solution de 13,5 g de chloro-4 (isopropyl-4 phénylamino)-5 dithiole-1,2 one-3 dans 165 cm³ de pyridine, on ajoute en 10 min 31,2 g d'anhydride mixte acétique formique à une température voisine de 5 °C. Le mélange réactionnel est agité à cette température pendant 3 h puis est versé dans 1650 cm³ d'eau distillée. L'émulsion obtenue est extraite 2 fois par 500 cm³ au total de chlorure de méthylène. Les phases organiques sont réunies et lavées par 100 cm³ d'eau distillée, séchées sur sulfate de sodium, filtrées puis concentrées à sec sous pression réduite (2,7 kPa) à 60 °C. Le résidu obtenu est repris dans un mélange d'éther éthylique et d'éther isopropylique (50/50 en volumes); les cristaux obtenues sont séparés par filtration, puis recristallisés dans l'éther isopropylique. On obtient ainsi 8,9 g de chloro-4 (N-formyl N-[(isopropyl-4 phényl) amino]-5 dithiole-1,2 one-3 fondant à 72 °C.

### Exemple 39

On agite pendant 20 h à une température voisine de 20 °C une suspension de 13,8 g de carbonate de potassium dans une solution de 18,7 g de dichloro-4,5 dithiole-1,2 one-3, de 13,2 g d'isopropyl-4-phénol et de 0,5 g de diméthylamino-4 pyridine dans 150 cm³ de diméthylformamide. Le mélange réactionnel est ensuite versé dans 1500 cm³ d'une solution aqueuse à 5% de chlorure d'ammonium et l'émulsion obtenue est extraite 1 fois par 400 cm³ puis 3 fois 300 cm³ au total de chlorure de méthylène. Les phases organiques sont réunies et lavées 2 fois par 500 cm³ au total d'eau distillée, séchées sur sulfate de magnésium, filtrées puis concentrées à sec sous pression réduite (2,7 kPa) à 70 °C. Le résidu obtenu est dissous dans 100 cm³ de chlorure de méthylène et la solution obtenue est versée sur 650 g de gel de silice contenus dans une colonne de 5,5 cm de diamètre. On élue d'abord par 1400 cm³ de chlorure de méthylène et élimine l'éluat correspondant; on élue ensuite par 1200 _{CM}³ de chlorure de méthylène et concentre l'éluat correspondant à sec sous pression réduite (2,7 kPa) à 40 °C. On obtient ainsi, après recristallisation dans l'éther isopropylique, 11,7 g de chloro-4 (isopropypl-4 phénoxy)-5 dithiole-1,2 one-3 fondant à 94 °C.

### Exemple 40

A une solution de 18,8 g d'amino-3 pyridine et de 38,2 g de nitrilo-tris (propanol-2) dans 1000 cm³ d'acétone à une température voisine de 0 °C, on ajoute 37,4 g de dichloro-4,5 dithiole-1,2 one-3 et le mélange réactionnel est agité à une température voisine de 0 °C pendant 16 h. Le produit insoluble est séparé par filtration et lavé 2 fois par 100 cm³ au total d'acétone. Les phases organiques sont réunies, séchées sur sulfate de magnésium, filtrées et concentrées à sec sous pression réduite (2,7 kPa) à 40 °C. Le résidu obtenu est introduit sur 7.50 g de gel de silice contenus dans une colonne de 6,5 cm de diamètre. On élue d'abord par 2,4 litres d'un mélange d'acétate d'éthyle et de méthanol (95/5 en volumes) et l'éluat correspondant est éliminé. On élue ensuite par 800 cm³ d'un mélange d'acétate d'éthyle et de méthanol (95/5 en volumes) et concentre l'éluat correspondant à sec sous pression réduite (2,7 kPa) à 40 °C. Le résidu obtenu est repris avec 500 cm³ d'acétonitrile et le produit insoluble est séparé par filtration. Après recristallisation dans 470 cm³ de méthylisobutylcétone, on obtient 4,65 g de chloro-4 (pyridyl-3 amino)-5 dithiole-1,2 one-3 fondant à 235 °C.

### Exemple 41

A une solution de 28,2 g d'amino-2 pyridine, de 57,3 g de nitrilo-tris (propanol-2) et de 1,5 g de diméthylamino-4 pyridine dans 1200 cm³ d'acétone à une température voisine de 0 °C, on ajoute sous agitation 56,1 g de dichloro-4,5 dithiole-1,2 one-3. Le mélange réactionnel est ensuite agité à une température voisine de 0°C pendant 17 h puis le produit insoluble formé est séparé par filtration et lavé 2 fois par 100 cm³ au total d'aétone. Les phases organiques sont réunies, séchées sur sulfate de magnésium filtrées et concentrées à sec sous pression réduite (2,7 kPa) à 40 °C. Le résidu obtenu est introduit sur 1500 g de gel de silice contenus dans une colonne de 10 cm de diamètre. On élue d'abord par 12,6 I de chlorure de méthylène; l'éluat correspondant est éliminé. On élue ensuite par 1200 cm³ d'un mélange de chlorure de méthylène et d'acétate d'éthyle (90/10 en volumes); l'éluat correspondant est concentré à sec sous pression réduite (2,7 kPa) à 40 °C. Par recristallisation du résidu obtenu dans 170 cm³ d'acide acétique, on obtient 2,84 g de chloro-4 (pyridyl-2 amino)-5 dithiole-1,2 one-3 fondant à 248 °C.

### Exemple 42

A une solution de 28,8 g d'amino-6 quinoléine et de 38,2 g de nitrilo-tris (propanol-2) dans 1000 cm³ d'acétone à une température voisine de 5 °C, on ajoute 37,4 g de dichloro-4,5 dithiole-1,2 one-3 et le mélange réactionnel est agité à une température voisine de 5 °C pendant 70 h. Le mélange réactionnel est ensuite évaporé à sec sous pression réduite (2,7 kPa) à 70 °C et le résidu est repris par un mélange de 2000 cm³ de chlorure de méthylène et de 2000 cm³ d'eau distillée. La suspension ainsi obtenue est agitée pendant 1 h à une température voisine de 20 °C et le produit insoluble restant est séparé par filtration. Le filtrat est décanté et la phase organique est lavée par 1500 cm³ d'eau distillée, séchée sur du sulfate de magnésium en présence de noir décolorant, filtrée et évaporée à sec sous pression réduite (2,7 kPa) à 40 °C. Le résidu ainsi obtenu est introduit sur 800 g de gel de silice contenus dans une colonne de 6 cm de diamètre. On élue d'abord par 7200 cm³ d'un mélange de chlorure de méthylène et d'acétate d'éthyle (90/10 en volumes) et élimine l'éluat correspondant. On élue ensuite par 2400 cm³ d'un mélange de chlorure de méthylène et d'acetate d'éthyle (75/25 en volumes) et élimine l'éluat correspondant. On élue ensuite par 16000 cm³ d'un mélange de chlorure de méthylène et d'acétate d'éthyle (60/40 en volumes); l'éluat correspondant est concentré à sec sous pression réduite (2,7 kPa) à 40 °C. Après recristallisation du résidu ainsi obtenu dans 3000 cm³ d'un mélange d'acétonitrile et de chlorure de méthylène (80/20 en volumes), on obtient 15 g de chloro-4 (quinolyl-6 amino)-5 dithiole-1,2 one-3 fondant à 232-4 °C.

### Exemple 43

A une solution de 28,8 g d'amino-5 isoquino- léine et de 38,2 g de nitrilo-tris (propanol-2) dans 1000 cm³ d'acétone à une température de 5 °C, on ajoute 37,4 g de dichloro-4,5 dithiole-1,2 one-3 et le mélange réactionnel est agité à une température voisine de 5 °C pendant 48 h. Le produit insoluble formé est séparé par filtration et lavé 3 fois par 600 cm³ au total d'acétone. Les filtrats organiques sont réunis et concentrés à sec sous pression réduite (2,7 kPa) à 40 °C. Le résidu ainsi obtenu est introduit sur 800 g de gel de silice contenus dans une colonne de 6 cm de diamètre. On élue d'abord par 11 d'un mélange de chlorure de méthylène et d'acétate d'éthyle (80/20 en volumes) et élimine l'éluat correspondant. On élue ensuite par 3,6 d'un mélange de chlorure de méthylène et d'acétate d'éthyle (80/20 en volumes) puis avec 1800 cm³ d'un mélange de chlorure de méthylène et d'acétate d'éthyle (70/30 en volumes) et enfin avec 6,4 I d'un mélange de chlorure de méthylène et d'acétate d'éthyle (50/50 en volumes); les éluats correspondants sont réunis et concentrés à sec sous pression réduite (2,7 kPa) à 40 °C. Après recristallisation du résidu obtenu dans 900 cm³ d'acide acétique, on obtient 10,5 g de chloro-4 (isoquinolyl-5 amino)₋5 dithiole-1,2 one-3 fondant à 251-3 °C avec décomposition.

### Exemple 44

A une solution de 18,8 g d'amino-8 quinoléine et de 24,8 g de nitrilo-tris (propanol-2) dans 650 cm³ d'acétone à une température voisine de 6 °C, on ajoute 24,4 g de dichloro-4,5 dithiole-1,2 one-3 et le mélange réactionnel est agité à une température voisine de 5 °C pendant 24 h. Le produit insoluble formé est ensuite séparé par filtration, lavé par 50 cm³ d'acétone est séché à l'air à une température voisine de 20 °C. Ce produit est ensuite repris par un mélange de 1000 cm³ d'éthanol et de 2000 cm³ de chlorure de méthylène et la suspension obtenue est agitée à une température voisine de 20 °C pendant 1 h. Le produit insoluble est séparé par filtration et séché à l'air à une température voisine de 20 °C. Après recristallisation dans 900 cm³ d'acide acétique, on obtient 10,0 g de chloro-4 (quinolyl-8 amino)-5 dithiole-1,2 one-3 fondant à 230 °C.

### Exemple 45

A une solution de 34 g d'amino-5 quinoléine, de 45 g de nitrilo-tris-(propanol-2) et de 1 g de diméthylamino-4 pyridine dans 1000 cm³ d'acétone à une température de 0 °C, on ajoute 44,1 g de dichloro-4,5 dithiole-1,2 one-3 et le mélange réactionnel est agité à une température voisine de 0 °C pendant 88 h. Le produit insoluble formé est ensuite séparé par filtration et lavé 3 fois avec 15 cm³ au total d'acétone. Le produit ainsi obtenu est introduit sur 1500 g de gel de silice contenus dans une colonne de 10 cm de diamètre. On élue d'abord par 8 1 d'un mélange de chlorure de méthylène et d'acétate d'éthyle (80/20 en volumes) et élimine l'éluat correspondant. On élue ensuite par 9 | d`un mélange de chlorure de méthylène et d'acétate d'éthyle (80/20 volumes) puis un mélange de chlorure de méthylène et d'acétate d'éthyle (40/60 en volumes) et les éluats correspondants sont réunis et concentrés à sec sous pression réduite (2,7 kPa) à 40 °C. Après recristallisation du résidu dans 220 cm³ d'acide acétique, on obtient 11,8 g de chloro-4 (quinolyl-5 amino)-5 dithiole-1,2 one-3 fondant à 241-2 °C avec décomposition.

### Exemple 46

A une solution de 8 g d'amino-5-indane et 11,2 g de dichloro-4,5 dithiole-1,2 one-3 dans 300 cm³ de méthanol, on ajoute 6,6 g de bicarbonate de sodium et le mélange réactionnel est agité pendant 24 h à une température voisine de 20 °C. Le produit insoluble formé est séparé par filtration et lavé par de l'eau distillée, jusqu'à disparition dans le filtrat aqueux des ions chlorure, puis séché à l'air à une température voisine de 20 °C. Le filtrat est concentré à sec sous pression réduite (2,7 kPa) à 40 °C et le résidu est repris avec 250 cm³ de chlorure de méthylène. La solution ainsi obtenue est lavée 4 fois par 600 cm³ au total d'eau distillée, séchée sur sulfate de magnésium en présence de noir décolorant, filtrée et concentrée à sec sous pression réduite (2,7 kPa) à 40 °C. Le résidu ainsi obtenu est réuni avec le produit insoluble séparé comme indiqué plus haut et recristallisé dans 190 cm³ d'acétonitrile. On obtient ainsi 14,0 g de chloro-4 (indanyl-5 amino)-5 dithiole-1,2 one-3 fondant à 150-2 °C.

### Exemple 47

A une solution de 17,4 g d'amino-6 quinoxaline et de 22,9 g de nitrilo-tris (propanol-2) dans 600 cm³ d'acétone à une température voisine de 5 °C, on ajoute 22,5 g de dichloro-4,5 dithiole-1,2 one-3 et le mélange réactionnel est agité à une température voisine de 5 °C pendant 48 h. Le mélange réactionnel est ensuite évaporé à sec sous pression réduite (2,7 kPa) à 70 °C et le résidu est lavé à l'eau distillée jusqu'à disparition de ions chlorure dans le filtrat puis séché à l'air à une température voisine de 20 °C. Ce produit est ensuite introduit sur 1000 g de gel de silice contenus dans une colonne de 8 cm de diamètre. On élue successivement par 6000 cm³ de chlorure de méthylène, 4200 cm³ d'un mélange de chlorure de méthylène et d'aétate d'éthyle (95/5 en volumes) 4000 cm³ d'un mélange de chlorure de méthylène et d'acétate d'éthyle (90/10 en volumes) 2000 cm³ d'un mélange de chlorure de méthylène et d'acétate d'éthyle (85/15 en volumes), 2250 cm³ d'un mélange de chlorure de méthylène et d'acétate d'éthyle (80/20 en volumes) et enfin 2000 cm³ d'un mélange de chlorure de méthylène et d'acétate d'éthyle (75/25 en volumes); tous les éluats correspondants sont éliminés. On élue ensuite par 7200 cm³ d'un mélange de chlorure de méthylène et d'acétate d'éthyle (60/40 en volumes) puis 2600 cm³ d'un mélange de chlorure de méthylène et d'acétate d'éthyle (50/50 en volumes). Les éluats correspondants sont réunis et concentrés à sec sous pression réduite (2,7 kPa) à 40 °C. Après recristallisation du résidu ainsi obtenu dans 400 cm³ d'acide acétique, on obtient 10 g de chloro-4 (quinoxalinyl-6 amino)-5 dithiole-1,2 one-3 fondant à 244 °C.

### Exemple 48

En opérant d'une manière analogue à celle décrite à l'exemple 1, mais à partir de 5,64 g de dichloro-4,5 dithiole-1,2 one-3 et de 6,7 g d'(amino-4 phényl)-2 butyramide, on obtient après recristallisation dans le méthanol, 2g de [(chloro-4 oxo-3 dithiole-1,2 yl-5) amino-4 phényl]-2 butyramide sous forme de cristaux jaune-pâle fondant à 196 °C.

L'(amino-4 phényl)-2 butyramide peut être préparée par une méthode analogue à celle décrite à l'exemple 32 pour la préparation de 1'(amino-4 phényl)-2 propionamide, mais à partir de 6,95 g de (nitro-4 phényl)-2 butyramide; on obtient ainsi 6,7 g. d'(amino-4 phényl)-2 butyramide sous forme d'une poudre blanche fondant à 126 °C.

La (nitro-4 phényl)-2 butyramide peut être préparée par une méthode analogue à celle décrite à l'exemple 32 pour la préparation de la (nitro-4 phényl)-2 propionamide, mais à partir de 19 g de (nitro-4 phényi)-2 butyronitrile; on obtient ainsi 6,95 g de (nitro-4 phényl)-2 butyramide sous forme de cristaux beiges fondant à 139 °C.

Le (nitro-4 phényi)-2 butyronitrile peut être préparé selon la méthode décrite par E. Fourneau et C. Sandulesco, Bull. Soc: Chim. France; 41, 450 (1927).

### Exemple 49

En opérant d'une manière analogue à celle décrite à l'exemple 1, mais à partir de 12,7 g de dichloro-4,5 dithiole-1,2 one-3 et de 10,3 g d'(amino-4 phényl) acétonitrile, on obtient, après recristallisation dans l'acide acétique, 12,7 g de [(chloro-4 oxo-3 dithiole-1,2 yl-5)-amino]-4 phénylacé- tamide sous forme de cristaux jaunes fondant à 230 °C.

L'(amino-4 phényl) acétonitrile peut être préparé par une méthode analogue à celle décrite à l'exemple 32 pour la préparation de 1'(amino-4 phényl)-2 propionamide, mais à partir de 18 g de (nitro-4 phényl) acétamide. On obtient 11 g d'(amino-4 phényl) acétamide sous forme de cristaux beiges fondant à 156 °C.

### Exemple 50

En opérant d'une manière analogue à celle décrite à l'exemple 1, mais à partir de 7,13 g de dichloro-4,5 dithiole-1,2 one-3 et de 6,26 g d'(amino-4 phényl)-3 propionamide, on obtient, après recristallisation dans le dioxanne, 2,85 g de [(chto- ro-4 oxo-3 dithiole-1,2 yl-5) amino-4 phényl]-3 propionamide sous forme d'une poudre jaune fondant à 197 °C.

L'(amino-4 phényl)-3 propionamide peut être préparée par hydrogénation catalytique de 30,4 g de nitro-4 cinnamamide dans 450 cm³ de méthanol en présence de 1,6 g de palladium à 5% sur noir de carbone à une température voisine de 30 °C sous une pression voisine de 100 kPa pendant 3 h. Après séparation du catalyseur par filtration, le mélange réactionnel est concentré à sec sous pression réduite (2,7 kPa) à 40 °C. On obtient ainsi 24,8 g d'(amino-4 phényl)-3 propionamide sous forme de cirstaux blancs fondant à 134 °C.

La nitro-4 cinnamamide peut être préparée selon la méthode décrite par L. Chiozza, Ann. Chim. Phys. [3] 39,196 (1853).

### Exemple 51

En opérant d'une manière analogue à celle décrite à l'exemple 1, mais à partir de 8,9 g de dichloro-4,5 dithiole-1,2 one-3 et de 8,5 g d'(amino-4 phényl)-2 N,N-diméthylacétamide, on obtient, après recristallisation dans un mélange d'acétate d'éthyle et d'éthanol (83/17 en volumes), 4,7 g de [(chloro-4 oxo-3 dithiole-1,2 yl-5) amino-4 phényl]-2 N,N-diméthylacétamide sous forme d'une poudre jaune fondant à 165 °C.

L'(amino-4 phényl)-2 N,N-diméthylacétamide peut être préparée d'une manière analogue à celle décrite à l'exemple 32 pour la préparation de l'(amino-4 phényl)-2 propionamide, mais à partir de 10,2 g de N,N-diméthyl (nitro-4 phényl)-2 acétamide; on obtient ainsi 8,6 g d'(amino-4 phényl)-2 N,N-diméthylacétamide sous forme d'une poudre brune fondant à 103 °C.

La N,N-diméthyl (nitro-4 phényl)-2 acétamide peut être préparée selon la méthode décrite par H.J. Taverne, Rec. Trav. Chim. des Pays-Bas, 16, 38 (1897).

### Exemple 52

En opérant d'une manière analogue à celle décrite à l'exemple 1, mais à partir de 11,4 g de dichloro-4,5 dithiole-1,2 one-3 et de 10,9 g d'(amino-4 phényl)-2 méthyl-2 propionamide, on obtient, après recristallisation dans l'acide acétique, 6,2 g de [(chloro-4 oxo-3 dithiole-1,2 yl-5) amino-4 phényl]-2 méthyl-2 propionamide sous forme d'une poudre jaune fondant à 193 °C.

L'(amino-4 phényl)-2 méthyl-2 propionamide peut être préparée par une méthode analogue à celle décrite à l'exemple 32 pour la préparation de 1'(amino-4 phényl)-2 propionamide, mais à partir de 28,4 g de méthyl-2 (nitro-4 phényl)-2 propionamide; on obtient ainsi 23,9 g d'(amino-4 phényl)-2 méthyl₋2 propionamide sous forme d'une poudre blanche fondant à 124 °C.

La méthyl-2 (nitro-4 phényl)-2 propionamide peut être préparée d'une manière analogue à celle décrite à l'exemple 32 pour la préparation de la (nitro-4 phényl)-2 propionamide, mais à partir de 39,3 g de méthyl-2 (nitro-4 phényl) propionitrile; on obtient ainsi 28,8 g de méthyl-2 (nitro-4 phényl)-2 propionamide sous forme de cristaux blancs fondant à 104 °C.

Le méthyl-2 (nitro-4 phényl) propionitrile peut être obtenue de la manière suivante: A 180 cm³ d'une solution aqueuse à 93% d'acide nitrique maintenue à une température voisine de 0 °C on ajoute goutte à goutte en 50 min environ 39,6 g de méthyl-2 phényl-2 propionitrile. Le mélange réactionnel est agité pendant 20 h à une température voisine de 20 °C, puis est versé dans 2 d'eau glacée; le précipité formé est séparé par filtration, lavé par 5 fois 100 cm³ d'eau distillée et séché; on obtient ainsi 40,5 g de méthyl-2 (nitro-4 phényl) propionitrile sous forme d'une poudre blanche fondant à 78 °C.

Le méthyl-2 phényl-2 propionitrile peut être préparé selon la méthode décrite par L.B. Taranko et R.H. Perry, Jr.; J. Org. Chem. 34, 226 (1969).

### Exemple 53

En opérant d'une manière analogue à celle décrite à l'exemple 39, mais à partir de 9 g de dichloro-4,5 dithiole-1,2 one-3 et de 7,3 g d'(hydroxy-4 phényl)-2 acétamide, on obtient après recristallisation dans l'acétonitrile 3,5 g de [(chloro-4 oxo-3 dithiole-1,2 yl-5) oxy-4 phényl]-2 acétamide sous forme d'une poudre jaune fondant à 168 °C.

L'(hydroxy-4 phényl)-2 acétamide peut être préparé selon la méthode décrite par H. Salkowski Chem. Ber., 22, 2137 (1889).

La présente invention concerne également les médicaments constitués par un produit de formule générale (I), sous forme libre ou, le cas échéant, sous forme de sels d'addition, avec un acide, de sels métalliques ou de sels d'addition avec une base pharmaceutiquement acceptables, à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale, rectale ou topique.

Comme compositions solides pour administration orale peuvent être utilisés des comprimés, pilules, poudres (notamment dans des capsules de gélatine ou des cachets) ou granulés. Dans ces compositions, le produit actif selon l'invention est mélangé à un ou plusieurs diluants inertes, tels que amidon, cellulose, saccharose, lactose ou silice. Ces compositions peuvent également comprendre des substances autres que les diluants, par exemple un. ou plusieurs lubrifiants tel que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier d'huile d'olive, des esters organiques injectables, par exemple l'oléate d'éthyle ou autres solvants organiques convenables. Ces compositions peuvent également contenir des adjuvants, en particulier des agents mouillants, isoto- nisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi- synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les produits selon l'invention sont particulièrement utiles dans le traitement de fond de la maladie rhumatismale. Les doses dépendent de l'effet recherché et de la durée du traitement; elles sont généralement comprises entre 50 et 1000 mg par jour par voie orale pour un adulte en une ou plusieurs prises.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent des compositions selon l'invention.

### Exemple A

On prépare, selon la technique habituelle, des comprimés dosés à 50 mg de produit actif ayant la composition suivante:
[(chloro-4 oxo-3 dithiole-1,2 yl-5) amino-4 phényl]-

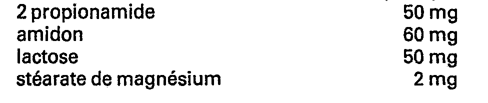

### Exemple B

On prépare une solution injectable contenant 20 mg de produit actif ayant la composition suivante:
Chlorhydrate d'[(amine-1 propyl-2)-4 phénvlami-

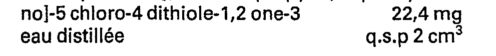

## Revendications (Revendications pour l'(les) Etat(s) contractant(s) suivant(s) : BE, CH, DE, FR, GB, IT, LI, LU, NL, SE)

1. Dérivé de la dithiole-1,2 one-3, caractérisé en ce qu'il répond à la formule générale: 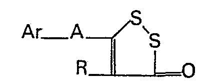 dans laquelle
- soit R représente un atome d'hydrogène ou de chlore, A représente un atome d'oxygène ou un radical imino, alcoylimino, alcoylcarbonylimino ou formylimino et Ar représente un radical phényle substitué par un radical cycloalcoyle dont la partie alcoyle contient 3 à 7 atomes de carbone, alcényle contenant 2 à 4 atomes de carbone en chaîne droite ou ramifiée, anilino, formyle, semicarbazonométhyle, dithiolanne-1,3 yle-2, dioxolanne-1,3 yle-2, dialcoylaminométhylèneamino, trifluorométhylthio, alcoylcarbonyloxy ou alcoyle substitué par un radical hydroxy, alcoyloxy, carboxy, amino, alcoylcarbonylamino, alcoyloxycarbonylamino, dialcoylaminométhy,lèneamino, formamido, alcoyluréido, phénylsulfonyle, carba- moyle, alcoylcarbamoyle, dialcoylcarbamoyle, formyle, semicarbazonométhyle, dithiolanne-1,3 yle-2 ou dioxolanne-1,3 yle-2 ou bien Ar représente un radical pyridyle, quinolyle, isoquinolyle, indanyle ou quinoxalinyle
- soit R représente un atome d'hydrogène, A représente un atome d'oxygène ou un radical imino, alcoylimino, alcoylcarbonylimino ou formylimino et Ar représente un radical phényle substitué par un radical alcoyle,
-soit R représente un atome de chlore, A représente un atome d'oxygène ou radical alcoylcarbonylimino ou formylimino et Ar représente un radical phényle substitué par un radical alcoyle, étant entendu que tous les radicaux alcoyle et portions alcoyle. contiennent, sauf mention spéciale, 1 à 4 atomes de carbone en chaîne droite ou ramifiée, ainsi que, lorsqu'ils existent, ses sels d'addition avec les acides, ses sels métalliques et ses sels d'addition avec les bases organiques.

2.. Procédé de préparation d'un produit selon la revendication 1 dans la formule duquel A représente un atome d'oxygène ou un radical imino ou alcoylimino, R représente un atome de chlore et Ar est défini comme à la revendication 1 à l'exception de représenter un radical phényle substitué par un radical dialcoylaminométhylèneamino ou un radical alcoyle -substitué par un radical dialcoylaminométhylèneamino, caractérisé en ce que l'on fait réagir un produit de formule géné- rate: 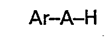 dans laquelle Ar et A ont les définitions correspondantes sur la dichloro-4,5 dithiole-1,2 one-3 de formule: 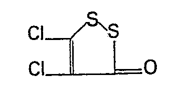 puis isole le produit obtenu et le transforme le cas échéant, en un sel d'addition avec un acide, en un sel métallique ou en un sel d'addition avec une base azotée.

3. Procédé de préparation d'un produit selon la revendication 1 dans la formule duquel A représente un radical alcoylimino et les autres symboles sont définis comme à la revendication 1, caractérisé en ce que l'on fait réagir un produit de formule générale: 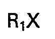 dans laquelle R₁ représente un radical alcoyle selon la revendication 1 et X représente un atome d'halogène ou un reste d'ester réactif tel que mésyloxy ou tosyloxy, sur un produit selon la revendication 1 dans la formule duquel A représente un radical imino et les autres symboles sont définis comme à la revendication 1, c'est-à-dire un produit de formule générale: 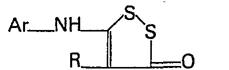 puis isole le produit obtenu et le transforme, le cas échéant, en un sel d'addition avec un acide, en un sel métallique ou en un sel d'addition avec une base organique.

4. Procédé de préparation d'un produit selon la revendication 1 dans la formule duquel A représente un radical alcoylcarbonylimino ou formylimino et les autres symboles sont définis comme à la revendication 1, caractérisé en ce que l'on fait réagir un anhydride mixte de formule générale: 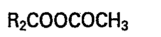 dans laquelle R₂ représente un atome d'hydrogène ou un radical alcoyle selon la revendication 1, sur un produit selon la revendication 1 dans la formule duquel A réprésente un radical imino et les autres symboles sont définis à la revendication 1, c'est-à-dire un produit de formule générale: 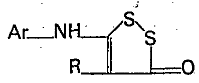 puis isole le produit obtenu et le transforme, le cas échéant, en un sel d'addition avec un acide en un sel métallique ou en un sel d'addition avec une base azotée.

5. Procédé de préparation d'un produit selon la revendication 1 dans la formule duquel R représente un atome d'hydrogène et les autres symboles sont définis comme à la revendication 1, caractérisé en ce que l'on déchlore un produit selon la revendication 1 dans la formule duquel R représente un atome de chlore et les autres symboles sont définis comme à la revendication 1, c'est-à-dire un produit de formule générale: 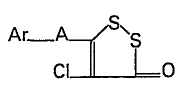 puis isole le produit obtenu et le transforme, le cas échéant, en un sel d'addition avec un acide, en un sel métallique ou en un sel d'addition avec une base azotée.

6. Procédé selon la revendication 5, caractérisé en ce que la déchloration s'effectue au moyen d'un hydrure de trialcoylétain.

7. Procédé selon l'une des revendications 5 ou 6, caractérisé en ce que la déchloration s'effectue avec l'hydrure de tributylétain.

8. Procédé de préparation d'un produit selon la revendication 1 dans la formule duquel Ar représente un radical phényle substitué par un radical dithiolanne-1,3 yle-2, dioxolanne-1,3 yle-2 ou alcoyle substitué par un radical dithiolanne-1,3 yle-2 ou dioxolanne-1,3 yle-2 et les autres symboles sont définis comme à la revendication 1, caractérisé en ce que l'on fait réagir un produit de formule générale: 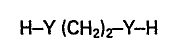 dans laquelle les symboles Y représentent tous les deux un atome de soufre ou d'oxygène, sur un produit selon la revendication 1 dans la formule duquel Ar représente un radical phényle substitué par un radical formyle ou alcoyle substitué par un radical formyle, c'est-à-dire un produit de formule générale: 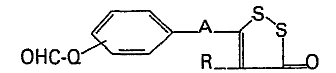 dans laquelle Q représente une liaison de valence ou un radical alcoylène contenant 1 à 4 atomes de carbone en chaîne droite ou ramifiée, et les autres symboles sont définis comme à la revendication 1, puis isole le produit obtenu.

9. Procédé de préparation d'un produit selon la revendication 1 dans la formule duquel Ar représente un radical phényle substitué par un radical dialcoylaminométhylèneamino ou alcoyle substitué par un radical dialcoylaminométhylèneamino et les autres symboles sont définis comme à la revendication 1, caractérisé en ce que l'on fait réagir un produit de formule générale: 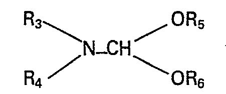 dans laquelle les radicaux R₃, R₄, R₅ et R₆ représentent des radicaux alcoyle selon la revendication 1, sur un produit selon la revendication 1 dans la formule duquel Ar représente un radical phényle substitué par un radical amino ou alcoyle substitué par un radical amino, c'est-à-dire un produit de formule générale: 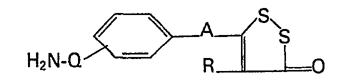 dans laquelle Q représente une liaison de valence ou un radical alcoylène contenant 1 à 4 atomes de carbone en chaîne droite ou ramifiée et les autres symboles sont définis comme à la revendication 1, puis isole le produit obtenu et le transforme, éventuellement, en un sel d'addition avec un acide.

10. Procédé de préparation d'un produit selon la revendication 1 dans la formule duquel Ar représente un radical phényle substitué par un radical alcoyle lui-même substitué par un radical alcoylcarbonylamino ou alcoyloxycarbonylamino et les autres symboles sont définis comme à la revendication 1, caractérisé en ce que l'on fait réagir un produit de formule générale: 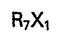 dans laquelle R₇ représente un radical alcoylcar- bonyle ou alcoyloxycarbonyle selon la revendication 1 et X₁ représente un atome d'halogène, sur un produit selon la revendication 1 dans la formule duquel Ar représente un radical phényle substitué par un radical alcoyle substitué par un radical amino et les autres symboles sont définis comme à la revendication 1, c'est-à-dire un produit de formule générale: 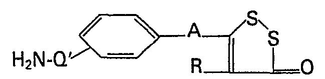 dans laquelle Q' représente un radical alcoylène contenant 1 à 4 atomes de carbone en chaîne droite ou ramifiée et les autres symboles sont définis comme à la revendication 1, puis isole le produit obtenu.

11. Procédé de préparation d'un produit selon la revendication 1 dans la formule duquel Ar représente un radical phényle substitué par un radical alcoyle substitué par un radical formamido et les autres symboles sont définis comme à la revendication 1, caractérisé en ce que l'on fait réagir un formiate d'alcoyle de formule générale: 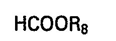 dans laquelle R₈ représente un radical alcoyle, sur un produit selon la revendication 1 dans la formule duquel Ar représente un radical phényle substitué par un radical alcoyle lui-même substitué par un radical amino, c'est-à-dire un produit de formule générale: 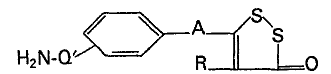 dans laquelle Q' représente un radical alcoylène contenant 1 à 4 atomes de carbone en chaîne droite ou ramifiée et les autres symboles sont définis comme à la revendication 1, puis isole le produit obtenu.

12. Procédé de préparation d'un produit selon la revendication 1 dans la formule duquel Ar représente un radical phényle substitué lui-même par un radical alcoyle substitué par un radical uréido et les autres symboles sont définis comme à la revendication 1, caractérisé en ce que l'on fait réagir un isocyanate de formule générale: 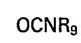 dans laquelle R₉ représente un radical alcoyle selon la revendication 1 sur un produit selon la revendication 1 dans la formule duquel Ar représente un radical phényle substitué par un radical alcoyle lui-même substitué par un radical amino, c'est-à-dire un produit de formule générale: 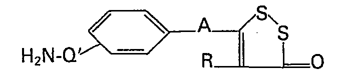 dans laquelle Q' représente un radical alcoylène contenant 1 à 4 atomes de carbone en chaîne droite ou ramifiée et les autres symboles sont définis comme à la revendication 1, puis isole le produit obtenu.

13. Composition pharmaceutique caractérisé en ce qu'elle contient au moins un produit selon la revendication 1 en association avec un ou plusieurs diluants ou adjuvants compatibles et pharmaceutiquement acceptables.

## Revendications (Revendications pour l'(les) Etat(s) contractant(s) suivant(s) : AT)

1. Procédé de préparation d'un dérivé de la dithiole-1,2-one-3 de formule générale: 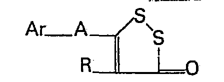 dans laquelle
-soit R représente un atome d'hydrogène ou de chlore, A représente un atome d'oxygène ou un radical imino, alcoylimino, alcoylcarbonylimino ou formylimino et Ar représente un radical phé- nylesubstitué par un radical cycloalcoyle dont la partie alcoyle contient 3 à 7 atomes de carbone, alcényle contenant 2 à 4 atomes de carbone en chaîne droite ou ramifiée, anilino, formyle, semicarbazonométhyle, dithiolanne-1,3 yle-2, dioxolanne-1,3 yle-2, dialcoylaminométhylèneamino, trifluorométhylthio, alcoylcarbonyloxy ou alcoyle substitué par un radical hydroxy, alcoyloxy, carboxy, amino, alcoylcarbonylamino, alcoyloxycar- bonylamind, dialcoylaminométhylèneamino, formamido, alcoyluréido, phénylsulfonyle, carba- moyle, alcoylcarbamoyle, dialcoylcarbamoyle, formyle, semicarbazonométhyle, dithiolanne-1,3 yle-2 ou dioxolanne-1,3 yle-2 ou bien Ar représente un radical pyridyle, quinolyle, isoquinolyle, indanyle ou quinoxalinyle,
- soit R représente un atome d'hydrogène, A représente un atome d'oxygène ou un radical imino, alcoylimino, alcoylcarbonylimino ou formylimino et Ar représente un radical phényle substitué par un radical alcoyle;
- soit R représente un atome de chlore, A représente un atome d'oxygène ou radical alcoylcarbonylimino ou formylimino et Ar représente un radical phényle substitué par un radical alcoyle, étant entendu que tous les radicaux alcoyle et portions alcoyle contiennent, sauf mention spéciale, 1 à 4 atomes de carbone en chaîne droite ou ramifiée, ainsi que, lorsqu'ils existent, ses sels d'addition avec les acides, ses sels métalliques et ses sels d'addition avec les bases organiques, caractérisé en ce que
a) pour la préparation d'un produit de formule générale (I) dans laquelle A représente un atome d'oxygène ou un radical imino ou alcoylimino, R représente un atome de chlore et Ar est défini comme précédemment à l'exception de représenter un radical phényle substitué par un radical dialcoylaminométhylèneamino ou un radical alcoyle substitué par un radical dialcoylaminométhylèneamino, on fait réagir un produit de formule générale: 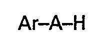 dans laquelle Ar et A ont les définitions correspondantes sur la dichloro-4,5 dithiole-1,2 one-3 de formule: 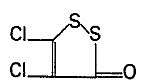 puis isole le produit obtenu et le transforme, le cas échéant, en un sel d'addition avec un acide, en un sel métallique ou en un sel d'addition avec une base azotée, ou en ce que
b) pour la préparation d'un produit de formule générale (I) dans laquelle A représente un radical alcoylimino et les autres symboles sont définis comme précédemment, on fait réagir un produit de formule générale: 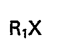 dans laquelle R₁ représente un radical alcoyle etX représente un atome d'halogène ou un reste d'ester réactif tel que mésyloxy ou tosyloxy, sur un produit de formule générale (I) dans laquelle A représente un radical imino et les autres symboles sont définis comme précédemment, c'est-à-dire un produit de formule générale: 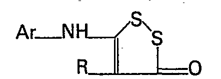 puis isole le produit obtenu et le transforme, le cas échéant, en un sel d'addition avec un acide, en un sel métallique ou en un sel d'addition avec une base organique, ou en ce que
c) pour la préparation d'un produit de formule générale (I) dans laquelle A représente un radical alcoylcarbonylimino ou formylimino et les autres symboles sont définis comme précédemment, on fait réagir un anhydride mixte de formule générale: 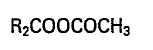 dans laquelle R₂ représente un atome d'hydrogène ou un radical alcoyle, sur un produit de formule générale (I) dans laquelle A représente un radical imino et les autres symboles sont définis comme précédemment, c'est-à-dire un produit de formule générale: 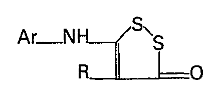 puis isole le produit obtenu et le transforme, le cas échéant, en un sel d'addition avec un acide, en un sel métallique ou en un sel d'addition avec une base azotée, ou en ce que
d) pour la préparation d'un produit de formule générale (I) dans laquelle R représente un atome d'hydrogène et les autres symboles sont definis comme précédemment, on déchlore un produit de formule générale (1) dans laquelle R représente un atome de chlore et les autres symboles sont définis comme précédemment, c'est-à-dire un produit de formule générale: 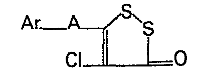 puis isole le produit obtenu et le transforme, le cas échéant, en un sel d'addition avec un acide, en un sel métallique ou en un sel d'addition avec une base azotée, ou en ce que
e) pour la préparation d'un produit de formule générale (I) dans laquelle Ar représente un radical phényle substitué par un radical dithiolanne-1,3 yle-2, dioxolanne-1,3 yle-2 ou alcoyle substitué par un radical dithiolanne-1,3 yle-2 ou dioxolanne-1,3 yle-2 et les autres symboles sont définis comme précédemment, on fait réagir un produit de formule générale: 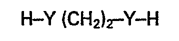 dans laquelle les symboles Y représentent tous les deux un atome de soufre ou d'oxygne, sur un produit de formule générale (I) dans laquelle Ar représente un radical phényle substitué par un radical formyle ou alcoyle substitué par un radical formyle, c'est-à-dire un produit de formule générale: 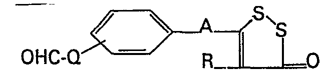 dans laquelle Q représente une liaison de valence ou un radical alcoylène contenant 1 à 4 atomes de carbone en chaîne droite ou ramifiée, et les autres symboles sont définis comme précédemment, puis isole le produit obtenu, ou en ce que
f) pour la préparation d'un produit de formule générale (I) dans laquelle Ar représente un radical phényle substitué par un radical dialcoylaminométhylèneamino ou alcoyle substitué par un radical dialcoylaminométhylèneamino et les autres symboles sont définis comme précédemment, on fait réagir un produit de formule générale: 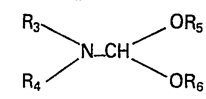 dans laquelle les radicaux R₃, R₄, R₅ et Rₑ représentent des radicaux alcoyle, sur un produit de formule générale (I) dans laquelle Ar représente un radical phényle substitué par un radical amino ou alcoyle substitué par un radical amino, c'est-à-dire un produit de formule générale: 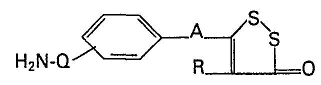 dans laquelle Q représente une liaison de valence ou un radical alcoylène contenant 1 à 4 atomes de carbone en chaîne droite ou ramifiée et les autres symboles sont définis comme précédemment, puis isole le produit obtenu et le transforme, éventuellement, en un sel d'addition avec un acide, ou en ce que
g) pour la préparation d'un produit de formule générale (I) dans laquelle Ar représente un radical phényle substitué par un radical alcoyle lui-même substitué par un radical alcoylcarbonylamino ou alcoyloxycarbonylamino et les autres symboles sont définis comme précédemment, on fait réagir un produit de formule générale: 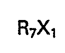 dans laquelle R₇ représente un radical alcoylcar- bonyle ou alcoyloxycarbonyle et X₁ représente un atome d'halogène, sur un produit de formule générale (I) dans laquelle Ar représente un radical phényle substitué par un radical alcoyle substitué par un radical amino et les autres symboles sont définis comme précédemment, c'est-à-dire un produit de formule générale: 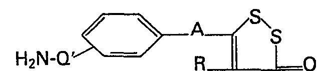 dans laquelle Q' représente un radical alcoylène contenant 1 à 4 atomes de carbone en chaîne droite ou ramifiée et les autres symboles sont définis comme précédemment, puis isole le produit obtenu, ou en ce que
h) pour la préparation d'un produit de formule générale (I) dans laquelle Ar représente un radical phényle substitué par un radical alcoyle substitué par un radical formamido et les autres symboles sont définis comme précédemment, on fait réagir un formiate d'alcoyle de formule générale: 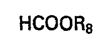 dans laquelle Rε représente un radical alcoyle, sur un produit de formule générale (I) dans laquelle Ar représente un radical phényle substitué par un radical alcoyle lui-même substitué par un radical amino, c'est-à-dire un produit de formule générale: 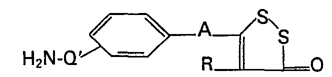 dans laquelle Q' représente un radical alcoylène contenant 1 à 4 atomes de carbone en chaîne droite ou ramifiée et les autres symboles sont définis comme précédemment, puis isole le produit obtenu, ou en ce que
i) pour la préparation d'un produit de formule générale (I) dans laquelle Ar représente un radical phényle substitué lui-même pour un radical alcoyle substitué par un radical uréido et les autres symboles sont définis comme précédemment, on fait réagir un isocyanate de formule générale: 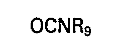 dans laquelle R₉ représente un radical alcoyle sur un produit de formule générale (I) dans laquelle Ar représente un radical phényle substitué par un radical alcoyle lui-même substitué par un radical amino, c'est-à-dire un produit de formule générale: 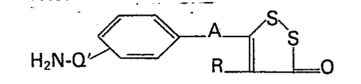 dans laquelle Q' représente un radical alcoylène contenant 1 à 4 atomes de carbone en chaîne droite ou ramifiée et les autres symboles sont définis comme précédemment, puis isole le produit obtenu.

2. Procédé selon la revendication 1 d) caractérisé en ce que la déchloration s'effectue au moyen d'un hydrure de trialcoylétain.

3. Procédé selon l'une des revendications 1d) ou 2, caractérisé en ce que la déchloration s'effectue avec l'hydrure de tributylétain.

## Patentansprüche (Patentansprüche für folgende(n) Vertragsstaat(en) : BE, CH, DE, FR, GB, IT, LI, LU, NL, SE)

1. Derivat des 1,2-Dithiol-3-ons, dadurch gekennzeichnet, dass es der allgemeinen Formel 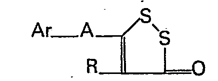 entspricht, worin
R entweder ein Wasserstoff- oder Chloratom bedeutet, A ein Sauerstoffatom oder einen lmino-, Alkylimino-_{;} Alkylcarbonylimino- oder Formyliminorest darstellt und-Ar einen Phenylrest bedeutet, substituiert durch einen Cycloalkylrest, dessen Alkylteil 3 bis 7 Kohlenstoffatome enthält, Alkenyl mit 2 bis 4 Kohlenstoffatomen in gerader oder verzweigter Kette, Anilino, Formyl, Semicarbazonomethyl, 1,3-Dithiolan-2-yl, 1,3-Dioxolan-2-yl, Dialkylaminomethylenamino, Trifluormethylthio, Alkylcarbonyloxy oder Alkyl, substituiert durch einen Rest Hydroxy, Alkyloxy, Carboxy, Amino, Alkylcarbonylamino, Alkyloxycarbonylamino, Dialkylaminomethylenamino, Formamido, Alkylureido, Phenylsulfonyl, Carbamoyl, Alkylcarbamoyl, Dialkylcarbamoyl, Formyl, Semicarbazonomethyl, 1,3-Dithiolan-2-yl oder 1,3-Dioxolan-2-yl, oder Ar bedeutet einen Rest Pyridyl, Chinolyl, Isochinolyl, Indanyl oder Chinoxalinyl,
oder R ein Wasserstoffatom bedeutet, A ein Sauerstoffatom oder einen Imino-, Alkylimino-, Alkylcarbonylimino- oder Formyliminorest bedeutet und Ar einen Phenylrest, substituiert durch einen Alkylrest, darstellt,
oder R ein Chloratom bedeutet, A ein Sauerstoffatom oder einen Alkylcarbonylimino- oder Formyliminorest darstellt und Ar einen Phenylrest bedeutet, substituiert durch einen Alkylrest, wobei alle Alkylreste und Alkylteile ausser spezieller Erwähnung 1 bis 4 Kohlenstoffatome in gerader oder verzweigter Kette enthalten, sowie, falls sie existieren, ihre Additionssalze mit Säuren, ihre Metallsalze und ihre Additionssalze mit organischen Basen.

2. Verfahren zur Herstellung eines Produktes gemäss Anspruch 1, in dessen Formel A ein Sauerstoffatom oder einen lmino- oder Alkyliminorest bedeutet, R ein Chloratom darstellt und Ar wie in Anspruch 1 definiert ist, mit Ausnahme der Bedeutung eines Phenylrestes, substituiert durch einen Dialkylaminomethylenaminorest, oder eines Alkylrestes, substituiert durch einen Dialkylaminomethylenaminorest, dadurch gekennzeichnet, dass man ein Produkt der allgemeinen Formel 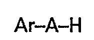 worin Ar und A die entsprechenden Bedeutungen haben, auf 4,5-Dichlor-1,2-dithiol-3-on der Formel 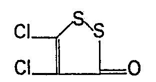 einwirken lässt, das erhaltene Produkt dann isoliert und es gegebenenfalls in ein Additionssalz mit einer Säure, in ein Metallsalz oder in ein Additionssalz mit einer Stickstoffbase überführt.

3. Verfahren zur Herstellung eines Produktes gemäss Anspruch 1, in dessen Formel A einen Alkyliminorest bedeutet und die übrigen Symbole wie in Anspruch 1 definiert sind, dadurch gekennzeichnet, dass man ein Produkt der allgemeinen Formel 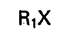 worin R₁ einen Alkylrest gemäss Anspruch 1 bedeutet und X ein Halogenatom oder einen Rest eines reaktionsfähigen Esters, wie Mesyloxy oder Tosyloxy, darstellt, auf ein Produkt gemäss Anspruch 1, in dessen Formel A einen Iminorest bedeutet und die übrigen Symbole wie in Anspruch 1 definiert sind, d. h. ein Produkt der allgemeinen Formel 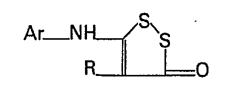 einwirken lässt, das erhaltene Produkt dann isoliert und es gegebenenfalls in ein Additionssalz mit einer Säure, in ein Metallsalz oder in ein Additionssalz mit einer organischen Base überführt.

4. Verfahren zur Herstellung eines Produkts gemäss Anspruch 1, in dessen Formel A einen Alkylcarbonylimino- oder Formyliminorest bedeutet und die übrigen Symbole wie in Anspruch 1 definiert sind, dadurch gekennzeichnet, dass man ein gemischtes Anhydrid der allgemeinen Formel 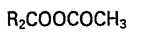 worin R₂ ein Wasserstoffatom oder einen Alkylrest gemäss Anspruch 1 bedeutet, auf ein Produkt gemäss Anspruch 1, in dessen Formel A einen Iminorest bedeutet und die übrigen Symbole wie in Anspruch 1 definiert sind, d. h. ein Produkt der allgemeinen Formel 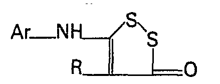 einwirken lässt, das erhaltene Produkt dann isoliert und es gegebenenfalls in ein Additionssalz mit einer Säure, in ein Metallsalz oder in ein Additionssalz mit einer Stickstoffbase überführt.

5. Verfahren zur Herstellung eines Produkts gemäss Anspruch 1, in dessen Formel R ein Wasserstoffatom bedeutet und die übrigen Symbole wie in Anspruch 1 definiert sind, dadurch gekennzeichnet, dass man ein Produkt gemäss Anspruch 1, in dessen Formel R ein Chloratom bedeutet und die übrigen Symbole wie in Anspruch 1 definiert sind, d. h. ein Produkt der allgemeinen Formel 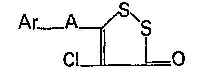 dechloriert, das erhaltene Produkt dann isoliert und es gegebenenfalls in ein Additionssalz mit einer Säure, in ein Metallsalz oder in ein Additionssalz mit einer Stickstoffbase überführt.

6. Verfahren gemäss Anspruch 5, dadurch gekennzeichnet, dass die Dechlorierung mit einem Trialkyl-zinnhydrid durchgeführt wird.

7. Verfahren gemäss einem der Ansprüche 5 oder 6, dadurch gekennzeichnet, dass die Dechlorierung mit Tributylzinnhydrid bewirkt wird.

8. Verfahren zur Herstellung eines Produktes gemäss Anspruch 1, in dessen Formel Ar einen Phenylrest, substituiert durch einen 1,3-Dithiolan-yl-2, 1,3-Dioxolan-yl-2-Rest oder Alkylrest, substituiert durch einen 1,3-Dithiolan-yl-2 oder 1,3-Dioxolan-yl-2-Rest, bedeutet und die übrigen Symbole wie in Anspruch 1 definiert sind, dadurch gekennzeichnet, dass man ein Produkt der allgemeinen Formel 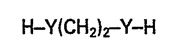 worin die Symbole Y alle beide ein Schwefel- oder Sauerstoffatom bedeuten, auf ein Produkt gemäss Anspruch 1, in dessen Formel Ar einen Phenylrest, substituiert durch einen Formylrest, oder Alkyl, substituiert durch einen Formylrest, bedeutet, d. h. ein Produkt der allgemeinen Formel 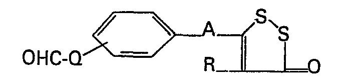 einwirken lässt, worin Q eine Valenzbindung oder einen Alkylenrest mit 1 bis 4 Kohlenstoffatomen in gerader oder verzweigter Kette bedeutet und die übrigen Symbole wie in Anspruch 1 definiert sind, und dann das erhaltene Produkt isoliert.

9. Verfahren zur Herstellung eines Produktes gemäss Anspruch 1, in dessen Formel Ar einen Phenylrest, substituiert durch einen Dialkylaminomethylenaminorest, oder Alkylrest, substituiert durch einen Dialkylaminomethylenaminorest, bedeutet und die übrigen Symbole wie in Anspruch 1 definiert sind, dadurch gekennzeichnet, dass man ein Produkt der allgemeinen Formel 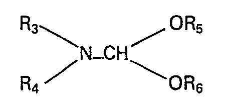 worin die Reste R₃, R₄, R₅ und R₆ Alkylreste gemäss Anspruch 1 bedeuten, auf ein Produkt gemäss Anspruch 1, in dessen Formel Ar einen Phenylrest, substituiert durch einen Aminorest, oder einen Alkylrest, substituiert durch einen Aminorest, bedeutet, d. h. ein Produkt der allgemeinen Formel 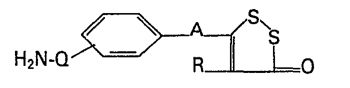 einwirken lässt, worin Q eine Valenzbindung oder einen Alkylenrest mit 1 bis 4 Kohlenstoffatomen in gerader oder verzweigter Kette bedeutet und die übrigen Symbole wie in Anspruch 1 definiert sind, dass man dann das erhaltene Produkt isoliert und es gegebenenfalls in ein Additionssalz mit einer Säure überführt.

10. Verfahren zur Herstellung eines Produkts gemäss Anspruch 1, in dessen Formel Ar einen Phenylrest bedeutet, substituiert durch einen Alkylrest, der seinerseits durch einen Alkylcarbonylamino- oder Alkyloxycarbonylaminorest substituiert ist, und die übrigen Symbole wie in Anspruch 1 definiert sind, dadurch gekennzeichnet, dass man ein Produkt der allgemeinen Formel 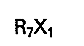 worin R₇ einen Alkylcarbonyl- oder Alkyloxycarbonylrest gemäss Anspruch 1 bedeutet und X₁ ein Halogenatom darstellt, auf ein Produkt gemäss Anspruch 1, in dessen Formel Ar einen Phenylrest bedeutet, der durch einen Alkylrest, substituiert durch einen Aminorest, substituiert ist, und die übrigen Symbole wie in Anspruch 1 definiert sind, d. h. ein Produkt der allgemeinen Formel 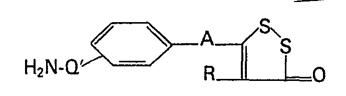 einwirken lässt, worin Q' einen Alkylenrest mit 1 bis 4 Kohlenstoffatomen in gerader oder verzweigter Kette bedeutet und die übrigen Symbole wie in Anspruch 1 definiert sind, und dass man das erhaltene Produkt dann isoliert.

11. Verfahren zur Herstellung eines Produkts gemäss Anspruch 1, in dessen Formel Ar einen Phenylrest, substituiert durch einen Alkylrest, substituiert durch einen Formamidorest, bedeutet und die übrigen Symbole wie in Anspruch 1 definiert sind, dadurch gekennzeichnet, dass man ein Alkylformiat der allgemeinen Formel 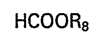 worin R₈ einen Alkylrest bedeutet, auf ein Produkt gemäss Anspruch 1, in dessen Formel Ar einen Phenylrest, substituiert durch einen Alkylrest, der seinerseits durch einen Aminorest substituiert ist, bedeutet, d. h. ein Produkt der allgemeinen Formel 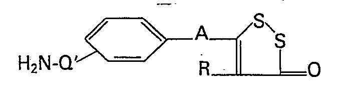 einwirken lässt, worin Q' einen Alkylenrest mit 1 bis 4 Kohlenstoffatomen in gerader oder verzweigter Kette bedeutet und die übrigen Symbole wie in Anspruch 1 definiert sind, und das erhaltene Produkt dann isoliert.

12. Verfahren zur Herstellung eines Produktes gemäss Anspruch 1, in dessen Formel Ar einen Phenylrest bedeutet, der seinerseits durch einen Alkylrest, substituiert durch einen Ureidorest, substituiert ist, und die übrigen Symbole wie in Anspruch 1 definiert sind, dadurch gekennzeichnet, dass man ein Isocyanat der allgemeinen Formel 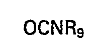 worin R₉ einen Alkylrest gemäss Anspruch 1 bedeutet, auf ein Produkt gemäss Anspruch 1, in dessen Formel Ar einen Phenylrest, substituiert durch einen Alkylrest, der seinerseits durch einen Aminorest substituiert ist, bedeutet, d. h. ein Produkt der allgemeinen Formel 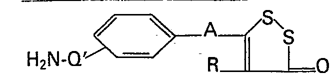 einwirken lässt, worin Q' einen Alkylenrest mit 1 bis 4 Kohlenstoffatomen in gerader oder verzweigter Kette bedeutet und die übrigen Symbole wie in Anspruch 1 definiert sind, und dann das erhaltene Produkt isoliert.

13. Pharmazeutische Zusammensetzung, dadurch gekennzeichnet, dass sie mindestens ein Produkt gemäss- Anspruch 1 enthält, zusammen mit einem oder mehreren verträglichen und pharmazeutisch annehmbaren Verdünnungs- oder Hilfsmitteln.

## Patentansprüche (Patentansprüche für folgende(n) Vertragsstaat(en) : AT)

1. Verfahren zur Herstellung eines 1,2-Dithiol-3- on-derivats der allgemeinen Formel: 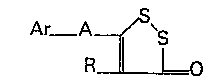 in welcher
- entweder R ein Wasserstoff- oder Chloratom darstellt, A ein Sauerstoffatom oder einen Imino-, Alkylimino-, Alkylcarbonylimino- oder Formyliminorest darstellt und Ar einen Phenylrest, substituiert durch einen Cycloalkylrest, dessen Alkylteil 3 bis 7 Kohlenstoffatome enthält, einen Alkenylrest, der 2 bis 4 Kohlenstoffatome in gerader oder verzweigter Kette enthält, einen Anilin-, Formyl-, Semicarbazonmethyl-, 1,3-Dithiolan-2-yl-, 1,3-Dioxolan-2-yl-, Dialkylaminomethylenamino-, Trifluormethylthio-, Alkylcarbonyloxyrest oder einen Alkylrest, substituiert durch einen Hydroxy-, Alkyloxy-, Carboxy-, Amino-, Alkylcarbonylamino-, Alkyloxycarbonylamino-, Dialkylaminomethylenamino-, Formamido-, Alkylureido-, Phenylsulfonyl-, Carbamoyl-, Alkylcarbamoyl-, Dialkylcarbamoyl-, Formyl-, Semicarbazonmethyl-, 1,3-Dithiolan-2-yl- oder 1,3-Dioxolan-2-yl-rest, darstellt, oder Ar für einen Pyridyl-, Chinolyl-, Isochinolyl-, Indanyl- oder Chinoxalinylrest steht,
- oder R ein Wasserstoffatom darstellt, A ein Sauerstoffatom oder einen Imino-, Alkylimino-, Alkylcarbonylimino- oder Formyliminorest darstellt und Ar einen durch einen Alkylrest substituierten Phenylrest darstellt,
- oder R ein Chloratom darstellt, A ein Sauerstoffatom oder einen Alkylcarbonylimino- oder Formyliminorest darstellt und Ar einen durch einen Alkylrest substituierten Phenylrest darstellt, wobei alle Alkylreste und Alkylteile ohne speziellen Hinweis selbstverständlich 1 bis 4 Kohlenstoffatome in gerader oder verzweigter Kette enthalten,
sowie, falls existent, von dessen Säureadditionssalzen, Metallsalzen und Additionssalzen mit organischen Basen, dadurch gekennzeichnet, dass man
a) zur Herstellung einer Verbindung der allgemeinen Formel (I), worin A ein Sauerstoffatom oder einen Imino- oder Alkyliminorest darstellt, R ein Chloratom darstellt und Ar die obige Bedeutung, ausgenommen einen Phenylrest, substituiert durch einen Dialkylaminomethylenamino-oder einen Alkylrest, substituiert durch einen Dialkylaminomethylenaminorest, hat, eine Verbindung der allgemeinen Formel: 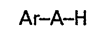 in welcher Ar und A die entsprechenden Bedeutungen haben, mit dem 4,5-Dichlor-1,2-dithiol-3- on der Formel: 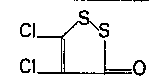 reagieren lässt, dann das erhaltene Produkt isoliert und es, zutreffendenfalls, in ein Säureadditionssalz, ein Metallsalz oder ein Additionssalz mit einer Stickstoffbase überführt, oder dass man
b) zur Herstellung einer Verbindung der allge₋ meinen Formel (I), worin A einen Alkyliminorest darstellt und die übrigen Symbole die obige Bedeutung haben, eine Verbindung der allgmeinen Formel: 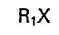 in welcher R₁ einen Alkylrest darstellt und X ein Halogenatom oder einen reaktionsfähigen Esterrest, z.8. Mesyloxy oder Tosyloxy, darstellt, mit einer Verbindung der allgemeinen Formel (I), in welcher A einen Iminorest darstellt und die übrigen Symbole die obige Bedeutung haben, d. h. einer Verbindung der allgemeinen Formel: 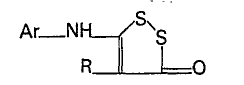 reagieren lässt und dann die erhaltene Verbindung isoliert und sie, zutreffendenfalls in ein Additionsalz mit einer Säure, ein Metallsalz oder ein Additionssalz mit einer organischen Base überführt, oder dass man
c) zur Herstellung einer Verbindung der allgemeinen Formel (1), in welcher A einen Alkylcarbonylimino- oder Formyliminorest darstellt und die übrigen Symbole die obige Bedeutung haben, ein gemischtes Anhydrid der allgemeinen Formel: 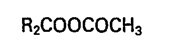 in welcher R₂ ein Wasserstoffatom oder einen Alkylrest darstellt, mit einer Verbindung der allgemeinen Formel (I), worin A einen Iminorest darstellt und die übrigen Symbole die obige Bedeutung haben, d. h. einer Verbindung der allgemeinen Formel: 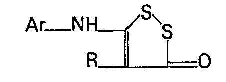 reagieren lässt und dann die erhaltene Verbindung isoliert und sie, zutreffendenfalls, in ein Additionssalz mit einer Säure, ein Metallsalz oder ein Additionssalz mit einer organischen Base überführt, oder dass man
d) zur Herstellung einer Verbindung der allgemeinen Formel (1), worin R ein Wasserstoffatom darstellt und die übrigen Symbole die obige Bedeutung haben, eine Verbindung der allgemeinen Formel (I), worin R ein Chloratom darstellt und die übrigen Symbole die obige Bedeutung haben, d. h. eine Verbindung der allgemeinen Formel: 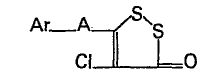 dechloriert und dann die erhaltene Verbindung isoliert und sie, zutreffendenfalls, in ein Additionssalz mit einer Säure, ein Metallsalz oder ein Additionssalz mit einer organischen Base überführt, oder dass man
e) zur Herstellung einer Verbindung der allgemeinen Formel (1), in welcher Ar einen Phenylrest, substituiert durch einen 1,3-Dithiolan-2-yl-, 1,3-Dioxolan-2-yl-Rest oder einen Alkylrest, substituiert durch einen 1,3-Dithiolan-2-yl- oder 1,3-Dioxolan-2-yl-Rest darstellt und die übrigen Symbole die obige Bedeutung haben, eine Verbindung der allgemeinen Formel: 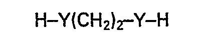 in welcher beide Symbole Y ein Schwefel- oder Sauerstoffatom darstellen, mit einer Verbindung der allgemeinen Formel (I), in welcher Ar einen Phenylrest, substituiert durch einen Formylrest oder einen Alkylrest, substituiert durch einen Formylrest, darstellt. d. h. einer Verbindung der allgemeinen Formel: 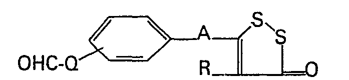 in welcher Q eine Valenzbindung oder einen Alkylenrest mit 1 bis 4 Kohlenstoffatomen in gerader oder verzweigter Kette darstellt und die übrigen Symbole die obige Bedeutung haben, reagieren lässt und dann die erhaltene Verbindung isoliert, oder dass man
f) zur Herstellung einer Verbindung der allgemeinen Formel (1), in welcher Ar einen Phenylrest, substituiert durch einen Dialkylaminomethylenaminorest oder einen Alkylrest, substituiert durch einen Dialkylaminomethylenaminorest, darstellt und die übrigen Symbole die obige Bedeutung haben, eine Verbindung der allgemeinen Formel: 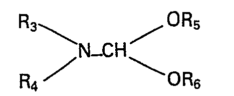 in welcher die Reste R₃, R₄, R₅ und R₆ Alkylreste darstellen, mit einer Verbindung der allgemeinen Formel (I), in welcher Ar einen Phenylrest, substituiert durch einen Aminorest oder einen Alkylrest, substituiert durch einen Aminorest, darstellt, d. h. einer Verbindung der allgemeinen Formel: 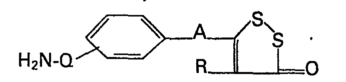 in welcher Q eine Valenzbindung oder einen Alkylenrest mit 1 bis 4 Kohlenstoffatomen in gerader oder verzweigter Kette darstellt und die übrigen Symbole die obige Bedeutung haben, reagieren lässt und dann die erhaltene Verbindung isoliert und sie, gegebenenfalls, in ein Additionssalz mit einer Säure überführt, oder dass man
g) zur Herstellung einer Verbindung der allgemeinen Formel (I), in welcher Ar einen Phenylrest, substituiert durch einen Alkylrest, der seinerseits durch einen Alkylcarbonylamino- oder Alkyloxycarbonylaminorest substituiert ist, darstellt und die übrigen Symbole die obige Bedeutung haben, eine Verbindung der allgemeinen Formel: 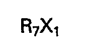 in welcher R₇ einen Alkylcarbonyl- oder Alkyloxycarbonylrest darstellt und X₁ ein Halogenatom darstellt, mit einer Verbindung der allgemeinen Formel (1), in welcher Ar einen Phenylrest, substituiert durch einen Alkylrest, substituiert durch einen Aminorest, darstellt und die übrigen Symbole die obige Bedeutung haben, d. h. einer Verbindung der allgemeinen Formel: 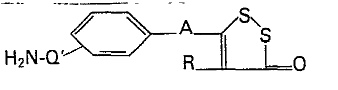 in welcher Q' einen Alkylenrest mit 1 bis 4 Kohlenstoffatomen in gerader oder verzweigter Kette darstellt und die übrigen Symbole die obige Bedeutung haben, reagieren lässt und dann die erhaltene Verbindung isoliert, oder dass man
h) zur Herstellung einer Verbindung der allgemeinen Formel (I), in welcher Ar einen Phenylrest, substituiert durch einen Alkylrest, substituiert durch einen Formamidorest, darstellt und die übrigen Symbole die obige Bedeutung haben, ein Alkylformiat der allgemeinen Formel 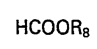 in welcher Rε einen Alkylrest darstellt, mit einer Verbindung der allgemeinen Formel (I), in welcher Ar einen Phenylrest, substituiert durch einen Alkylrest, der selbst durch einen Aminorest substituiert ist, d. h. einer Verbindung der allgemeinen Formel: 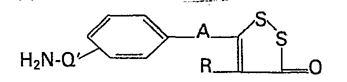 in welcher Q' einen Alkylenrest mit 1 bis 4 Kohlenstoffatomen in gerader oder verzweigter Kette darstellt und die übrigen Symbole die obige Bedeutung haben, reagieren lässt und dann die erhaltene Verbindung isoliert, oder dass man
i) zur Herstellung einer Verbindung der allgemeinen Formel (I), in welcher Ar einen Phenylrest, selbst substituiert durch einen Alkylrest, substituiert durch einen Ureidorest, darstellt und die übrigen Symbole die obige Bedeutung haben, mit einem Isocyanat der allgemeinen Formel: 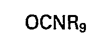 in welcher R₉ einen Alkylrest darstellt, mit einer Verbindung der allgemeinen Formel (1), in welcher Ar einen Phenylrest, substituiert durch einen Alkylrest, der seinerseits durch einen Aminorest substituiert ist; d. h. einer-Verbindung der allgemeinen Formel: - 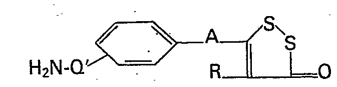 in welcher Q' einen Alkylenrest mit 1 bis 4 Kohlenstoffatomen in gerader oder verzweigter Kette darstellt und die übrigen Symbole die obige Bedeutung haben, reagieren lässt und dann die erhaltene Verbindung isoliert.

2. Verfahren nach Anspruch 1d), dadurch gekennzeichnet, dass die Dechlorierung mittels eines Trialkyletainhydrids erfolgt.

3. Verfahren nach einem der Ansprüche 1d) oder 2, dadurch gekennzeichnet, dass die Dechlorierung mittels Tributyletainhydrid erfolgt.

## Claims (Claims for the following Contracting State(s) : BE, CH, DE, FR, GB, IT, LI, LU, NL, SE)

1. 1,2-Dithiol-3-one derivative, characterized in that it corresponds to the general formula: 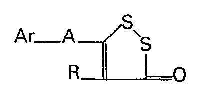 in which
- either R denotes a hydrogen or chlorine atom, A denotes an oxygen atom or an imino, alkylimino, alkylcarbonylimino or formylimino radical and Ar denotes a phenyl radical substituted by a cycloalkyl radical in which the alkyl part contains 3 to 7 carbon atoms, an alkenyl radical containing 2 to 4 carbon atoms as a straight or branched chain, an anilino, formyl, semicarbazonomethyl, 1,3-dithiolan-2-yl, 1,3-dioxolan-2-yl, dialkylamino- methyleneamino, trifluoromethylthio or alkylcarbonyloxy radical or an alkyl radical substituted by a hydroxy, alkoxy, carboxy, amino, alkylcarbonylamino, alkoxycarbonylamino, dialkylamino- methyleneamino, formamido, alkylureido, phenylsulphonyl, carbamoyl, alkylcarbamoyl, dialkylcarbamoyl, formyl, semicarbazonomethyl, 1,3-dithiolan-2-yl or 1,3-dioxolan-2-yi radical or Ar denotes a pyridyl, quinolyl, isoquinolyl, indanyl or quinoxalinyl radical,
- or R renotes a hydrogen atom, A denotes an oxygen atom or an imino, alkylimino, alkylcarbonylimino or formylimino radical and Ar denotes a phenyl radical substituted by an alkyl radical,
- or R denotes a chlorine atom, A denotes an oxygen atom or an alkylcarbonylimino or formylimino radical and Ar denotes a phenyl radical substituted by an alkyl radical, it being understood that, unless specially mentioned, all the alkyl radicals and alkyl moieties contain 1 to 4 carbon atoms as a straight or branched chain, as well as, where these exist, its salts of addition with acids, its metal salts and its salts of addition with organic bases.

2. Process for the preparation of a product according to claim 1, in the formula of which A denotes an oxygen atom or an imino or alkylimino radical, R denotes a chlorine atom and Ar is defined as in claim I except for denoting a phenyl radical substituted by a dialkylaminomethyleneamino radical or an alkyl radical substituted by a dialkylaminomethyleneamino radical, characterized in that a product of general formula: 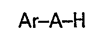 in which Ar and A have the corresponding definitions, is reacted with 4,5-dichloro-1,2-dithiol-3-one of formula: 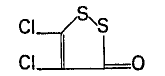 and the product obtained is then isolated and converted, if appropriate, into a salt of addition with an acid, into a metal salt or into a salt of addition with a nitrogenous base.

3. Process for the preparation of a product according to claim 1, in the formula of which A denotes an alkylimino radical and the other symbols are defined as in claim 1, characterized in that a product of general formula: 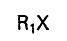 in which R_{I} denotes an alkyl radical according to claim 1 and X denotes a halogen atom or a reactive ester residue such as mesyloxy or tosyloxy, is reacted with a product according to claim 1, in the formula of which A denotes an imino radical and the other symbols are defined as in claim 1, that is to say a product of general formula: 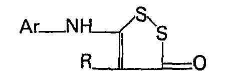 and the product obtained is then isolated and is converted, if appropriate, into a salt of addition with an acid, into a metal salt or into a salt of addition with an organic base.

4. Process for the preparation of a product according to claim 1, in the formula of which A denotes an alkylcarbonylimino or formylimino radical and the other symbols are defined as in claim 1, characterized in that a mixed anhydride of general formula: 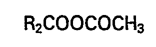 in which R₂ denotes a hydrogen atom or an alkyl radical according to claim 1, is reacted with a product according to claim 1, in the formula of which A denotes an imino radical and the other symbols are defined in claim 1, that is to say a product of general formula: 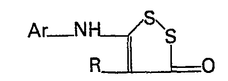 and the product obtained is then isolated and is converted, if appropriate, into a salt of addition with an acid, into a metal salt or into a salt of addition with a nitrogenous base.

5. Process for the preparation of a product according to claim 1, in the formula of which R denotes a hydrogen atom and the other symbols are defined as in claim 1, characterized in that a product according to claim 1, in the formula of which R denotes a chlorine atom and the other symbols are defined as in claim 1, that is to say a product of general formula: 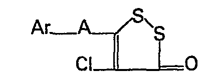 is dechlorinated and the product obtained is then isolated and is converted, if appropriate, into a salt of addition with an acid, into a metal salt or into a salt of addition with a nitrogenous base.

6. Process according to claim 5, characterized in that the dechlorination is carried out by means of a trialkyl in hydride.

7. Process according to either of claims 5 and 6, characterized in that the dechlorination is carried out with tributyl in hydride.

8. Process for the preparation of a product according to claim 1, in the formula of which Ar denotes a phenyl radical substituted by a 1,3-dithiolan-2-yl or 1,3-dioxolan-2-yl radical or an alkyl radical substituted by a 1,3-dithiolan-2-yl or 1,3-dioxolan-2-yl radical and the other symbols are defined as in claim 1, characterized in that a product of general formula: 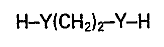 in which the symbols Y both denote a sulphur or oxygen atom, is reacted with a product according to claim 1, in the formula of which Ar denotes a phenyl radical substituted by a formyl radical or by an alkyl radical substituted by a formyl radical, that is to say a product of general formula: 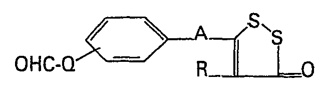 in which Q denotes a valency bond or an alkylene radical containing 1 to 4 carbon atoms as a straight or branched chain, and the other symbols are defined as in claim 1, and the product obtained is then isolated.

9. Process for the preparation of a product according to claim 1, in the formula of which Ar denotes a phenyl radical substituted by a dialkylaminomethyleneamino radical or by an alkyl radical substituted by a dialkylaminomethyleneamino radical and the other symbols are defined as in claim 1, characterized in that a product of general formula: 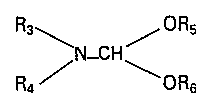 in which the radicals R₃, R₄, R₅ and R₆ denote alkyl radicals according to claim 1, is reacted with a product according to claim 1, in the formula of which Ar denotes a phenyl radical substituted by an amino radical or by an alkyl radical substituted by amino radical, that is to say a product of general formula: 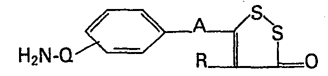 in which Q denotes a valency bond or an alkylene radical containing 1 to 4 carbon atoms as a straight or branched chain and the other symbols are defined as in claim 1, and the product obtained is then isolated and is converted, if appropriate, into a salt of addition with an acid.

10. Process for the preparation of a product according to claim 1, in the formula of which Ar denotes a phenyl radical substituted by an alkyl radical which is itself substituted by an alkylcarbonylamino or alkoxycarbonylamino radical and the other symbols are defined as in claim 1, characterized in that a product of general formula: 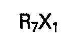 in which R₇ denotes an alkylcarbonyl or alkoxycarbonyl radical according to claim 1 and X, denotes a halogen atom, is reacted with a product according to claim 1, in the formula of which Ar denotes a phenyl radical substituted by an alkyl radical substituted by an amino radical and the other symbols are defined as in claim 1, that is to say a product of general formula: 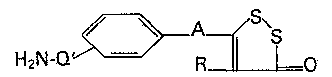 in which Q' denotes an alkylene radical containing 1 to 4 carbon atoms as a straight or branched chain and the other symbols are defined as in claim 1, and the product obtained is then isolated.

11. Process for the preparation of a product according to claim 1, in the formula of which Ar denotes a phenyl radical substituted by an alkyl radical substituted by a formamido radical and the other symbols are defined as in claim 1, characterized in that an alkyl formate of general formula: 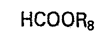 in which Rε denotes an alkyl radical, is reacted with a product according to claim 1, in the formula of which Ar denotes a phenyl radical substituted by an alkyl radical which is itself substituted by an amino radical, that is to say a product of general formula: 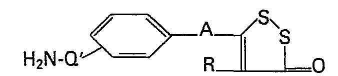 in which Q' denotes alkylene radical containing 1 to 4 carbon atoms as a straight or branched chain and the other symbols are defined as in claim 1, and the product obtained is then isolated.

12. Process for the preparation of a product according to claim 1, in the formula of which Ar denotes a phenyl radical which is itself substituted by an alkyl radical substituted by a ureido radical and the other symbols are defined as in claim 1, characterized in that an isocyanate of general formula: 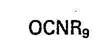 in which R₉ denotes an alkyl radical according to claim 1, is reacted with a product according to claim 1, in the formula of which Ar denotes a phenyl radical substituted by an alkyl radical which is itself substituted by an amino radical, that is to say a product of general formula: 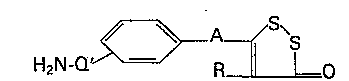 in which Q' denotes an alkylene radical containing 1 to 4 carbon atoms as a straight or branched chain and the other symbols are defined as in claim 1, and the product obtained is then isolated.

13. Pharmaceutical composition characterized in that it contains at least one product according to claim 1 in combination with one or more compatible and pharmaceutically acceptable diluents or adjuvants.

## Claims (Claims for the following Contracting State(s) : AT)

1. Process for the preparation of 1,2-dithiol-3- one derivative, of general formula: 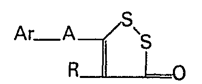 in which
- either R denotes a hydrogen or chlorine atom, A denotes an oxygen atom or an imino, alkylimino, alkylcarbonylimino or formylimino radical and Ar denotes a phenyl radical substituted by a cycloalkyl radical in which the alkyl part contains 3 to 7 carbon atoms, an alkenyl radical containing 2 to 4 carbon atoms as a straight or branched chain, an anilino, formyl, semicarbazonomethyl, 1,3-dithiolan-2-yl, 1,3-dioxolan-2-yl, dialkylamino- methyleneamino, trifluoromethylthio or alkylcarbonyloxy radical or an alkyl radical substituted by a hydroxy, alkoxy, carboxy, amino, alkylcarbonylamino, alkoxycarbonylamino, dialkylamino- methyleneamino, formamido, alkylureido, phenylsulphonyl, carbamoyl, alkylcarbamoyl, dialkylcarbamoyl, formyl, semicarbazonomethyl, 1,3-dithiolan-2-yl or 1,3-dioxolan-2-yl radical or Ar denotes a pyridyl, quinolyl, isoquinolyl, indanyl or quinoxalinyl radical,
- or R denotes a hydrogen atom, A denotes an oxygen atom or an imino, alkylimino, alkylcar, bonylimino or formylimino radical and Ar denotes a phenyl radical substituted by an alkyl radical,
- or R denotes a chlorine atom, A denotes an oxygen atom or an alkylcarbonylimino or formylimino radical and Ar denotes a phenyl radical substituted by an alkyl radical, it being understood that, unless specially mentioned, all the alkyl radicals and alkyl moieties contain 1 to 4 carbon atoms as a straight or branched chain, as well as, where these exist, its salts of addition with acids, its metal salts and its salts of addition with organic bases, characterized in that
a) for the preparation of a product of general formula (I) in which A denotes an oxygen atom or an imino or alkylimino radical, R denotes a chlorine atom and Ar is defined as above except for denoting a phenyl radical substituted by a dialkylamino-methyleneamino radical or an alkyl radical substituted by a dialkylaminomethyleneamino radical, a product of general formula: 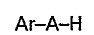 in which Ar and A have the corresponding definitions, is reacted with 4,5-dichloro-1,2-dithiol-3-one of formula: 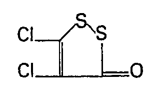 and the product obtained is then isolated and converted, if appropriate, into a salt of addition with an acid, into a metal salt or into a salt of addition with a nitrogenous base, or in that
b) for the preparation of a product of general formula (I) in which A denotes an alkylimino radical and the other symbols are defined as above, a product of general formula: 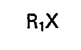 in which R₁ denotes an alkyl radical and X denotes a halogen atom or a reactive ester residue such as mesyloxy or tosyloxy, is reacted with a product of general formula (I), in which A denotes an imino radical and the other symbols are defined as above, that is to say a product of general formula: 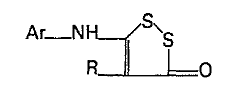 and the product obtained is then isolated and is converted, if appropriate, into a salt of addition with an acid, into a metal salt or into a salt of addition with an organic base, or in that
c) for the preparation of a product of general formula (I) in which A denotes an alkylcarbonylimino or formylimino radical and the other symbols are defined as above, a mixed anhydride of general formula: 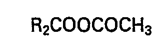 in which R₂ denotes a hydrogen atom or an alkyl radical, is reacted with a product of general formula (I) in which A denotes an imino radical and the other symbols are defined as above, that is to say a product of general formula: 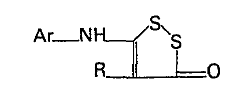 and the product obtained is then isolated and is converted, if appropriate, into a salt of addition with an acid, into a metal salt or into a salt of addition with a nitrogenous base, or in that,
d) for the preparation of a product of general formula (I) in which R denotes a hydrogen atom and the other symbols are defined as above, a product of general formula (I) in which R denotes a chlorine atom and the other symbols are defined as above, that is to say a product of general formula: 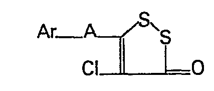 is dechlorinated and the product obtained is then isolated and is converted, if appropriate, into a salt of addition with an acid, into a metal salt or into a salt of addition with a nitrogenous base, or in that
e) for the preparation of a product of general formula (I), in which Ar denotes a phenyl radical substituted by a 1,3-dithiolan-2-yl or 1,3-dioxolan-2-yl radical or an alkyl radical substituted by a 1,3-dithiolan-2-yl or 1,3-dioxolan-2-yl radical and the other symbols are defined as above, a product of general formula: 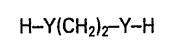 in which the symbols Y both denote a sulphur or oxygen atom, is reacted with a product of general formula (1), in which Ar denotes a phenyl radical substituted by a formyl radical or by an alkyl radical substituted by a formyl radical, that is to say a product of general formula: 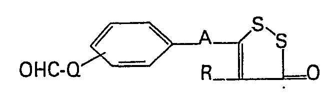 in which Q denotes a valency bond or an alkylene radical containing 1 to 4 carbon atoms as a straight or branched chain, and the other symbols are defined as above, and the product obtained is then isolated, or in that
f) for the preparation of a product of general formula (1), in which Ar denotes a phenyl radical substituted by a dialkylaminomethyleneamino radical or by an alkyl radical substituted by a dialkylaminomethyleneamino radical and the other symbols are defined as above, a product of general formula: 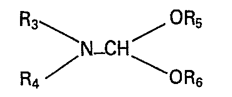 in which the radicals R₃, R₄, R₅ and R₆ denote alkyl radicals is reacted with a product of general formula (I), in which Ar denotes a phenyl radical substituted by an amino radical or by an alkyl radical substituted by amino radical, that is to say a product of general formula: 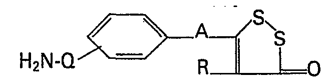 in which Q denotes a valency bond or an alkylene radical containing 1 to 4 carbon atoms as a straight or branched chain and the other symbols are defined as above, and the product obtained is then isolated and is converted, if appropriate, into a salt of addition with an acid, or in that
g) for the preparation of a product of general formula (I), in which Ar denotes a penyl radical substituted by an alkyl radical which is itself substituted by an alkylcarbonylamino or alkoxycarbonylamino radical and the other symbols are defined as above, a product of general formula: 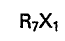 in which R₇ denotes an alkylcarbonyl or alkoxycarbonyl radical and X₁ denotes a halogen atom, is reacted with a product of general formula (I), in which A denotes a phenyl radical substituted by an alkyl radical substituted by an amino radical and the other symbols are defined as above, that is to say a product of general formula: 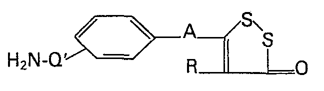 in which Q' denotes an alkylene radical containing 1 to 4 carbon atoms as a straight or branched chain and the other symbols are defined as above, and the product obtained is then isolated, or in that
h) for the preparation of a product of general formula (I), in which Ar denotes a phenyl radical substituted by an alkyl radical substituted by a formamido radical and the other symbols are defined as above, an alkyl formate of general formula: 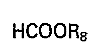 in which R₈ denotes an alkyl radical, is reacted with a product of general formula (I), in which Ar denotes a phenyl radical substituted by an alkyl radical which is itself substituted by an amino radical, that is to say a product of general formula: 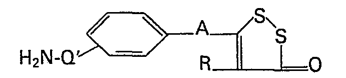 in which Q' denotes an alkylene radical containing 1 to 4 carbon atoms as a straight or branched chain and the other symbols are defined as above, and the product obtained is then isolated, or in that
i) for the preparation of a product of general formula (I), in which Ar denotes a phenyl radical which is itself substituted by an alkyl radical substituted by a ureido radical and the other symbols are defined as above, an isocyanate of general formula: 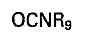 in which R₉ denotes an alkyl radical, is reacted with a product of general formula (I), in which Ar denotes a phenyl radical substituted by an alkyl radical which is itself substituted by an amino radical, that is to say a product of general formula: 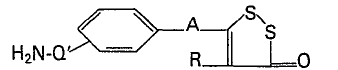 in which Q' denotes an alkylene radical containing 1 to 4 carbon atoms as a straight or branched chain and the other symbols are defined as above, and the product obtained is then isolated.

2. Process according to claim 1d), characterized in that the dechlorination is carried out by means of a trialkyltin hydride.

3. Process according to one of claims 1d) or 2, characterized in that the dechlorination is carried out by means of a tributyltin hydride.
